# EUROPEAN PATENT SPECIFICATION

(11) **EP 2 173 892 B1**
(45) Date of publication and mention of the grant of the patent: **21.09.2011**
(21) Application number: 07859596.4
(22) Date of filing: 27.07.2007
(51) Int. Cl.: C12P 35/04, C12P 37/04

(54) **Process for the preparation of penicillin or cephalosporin antibiotics**
Verfahren zur Herstellung von Penicillin- oder Cephalosporin-Antibiotika
Procédé de préparation d'antibiotiques pénicillines ou céphalosporines

(43) Date of publication of application: 14.04.2010
(73) Proprietor: Fermenta Biotech Limited, MAH (IN); Mikrobiologicky Ustac Avcr V.v.i., Praha 4- Krc - 142 20 (CZ)
(72) Inventor: DATLA, Anupama, Mumbai 400 049, Maharashtra (IN); RAJASEKAR, Vyasarayani, Williams, Thane (W) 400 610, Maharashtra (IN); KYSLIK, Pavel, Czech Republic (CZ); BECKA, Stanislav, Czech Republic (CZ); KRISHNAKANT, Ashar, Trupti, Thane (W) 400 607 Maharashtra (IN); YOGESH, Zambre, Sujata, Thane (W) 400 602 Maharashtra (IN); NIKUNJ, Kumar, Mumbai 400 019 Maharashtra (IN)
(74) Representative: Murray, Adrian D'Coligny
(86) International application number: PCT/IN2007/000319
(87) International publication number: WO 2009/016642

(56) References cited:
- WO-A-2004/050893
- US-B1- 6 403 356
- SKROB FRANTISEK ET AL: "Novel penicillin G acylase from Achromobacter sp. CCM 4824" ENZYME AND MICROBIAL TECHNOLOGY, STONEHAM, MA, US, vol. 32, no. 6, 20 May 2003 (2003-05-20), pages 738-744, XP002473789 ISSN: 0141-0229 cited in the application
- CAO L ET AL: "CROSS-LINKED AGGREGATES OF PENICILLIN ACYLASE: ROBUST CATALYSTS FOR THE SYNTHESIS OF BETA-LACTAM ANTIBIOTICS" JOURNAL OF MOLECULAR CATALYSIS. B, ENZYMATIC, ELSEVIER, AMSTERDAM, NL, vol. 4-06, no. 11, 22 January 2001 (2001-01-22), pages 665-670, XP001079507 ISSN: 1381-1177 cited in the application
- DE LEON A ET AL: "Periplasmic penicillin G acylase activity in recombinant Escherichia coli cells permeabilized with organic solvents." PROCESS BIOCHEMISTRY, vol. 39, no. 3, 28 November 2003 (2003-11-28), pages 301-305, XP008093218 ISSN: 1359-5113 cited in the application
- PRABHUNE A ET AL: "IMMOBILIZATION OF PENICILLIN ACYLASE IN POROUS BEADS OF POLYACRYLAMIDE GEL" APPLIED BIOCHEMISTRY AND BIOTECHNOLOGY, vol. 30, no. 3, 1991, pages 265-272, XP008093240 ISSN: 0273-2289

## Description

### Technical Field:

The present invention relates to isolation of Penicillin Acylase (PA) from *Achromobacter sp* CCM 4824 expressed in recombinant strain *E. coli BL21* CCM 7394 bearing the recombinant plasmid pKX1P1 and processing of PA into biocatalyst useful for the industrial synthesis of antibiotics. More particularly the invention relates to the synthesis of semi-synthetic β-lactam antibiotics from the reaction mixture consisting of activated acyl donor (D-p-hydroxyphenylglycine methyl ester or amide for Amoxicillin and Cefadroxil; D-phenylglycine methyl ester or amide for Ampicillin and Cephalexin) and nucleophile (6-APA or 7-ADCA) catalyzed by PA derived from recombinant *E.coli BL21* CCM 7394 as the biocatalyst.

### Background of the invention:

For several decades, Penicillin G acylase (E.C.3.5.1.11) has been commercially exploited to hydrolyse the acyl group of Penicillin G and its six member variant Deacetoxycephalosporin G, so as to yield 6-Aminopenicillanic acid (6-APA) and 7-Aminodeacetoxycephalosporanic acid (7-ADCA) respectively, the key building block for β-lactam antibiotics.

Synthetic properties of Penicillin acylase from *Escherichia coli ATCC 11105* are only moderate, which causes incomplete conversion of the subtrates to products. Similar Penicillin acylases are reported from *Kluyvera cryocrescens* and *Providencia rettgeri* but the synthetic potential of these enzymes are not well documented. In the recent years, a number of mutants with altered kinetic properties, especially with improved S/H ratios (the ratio of synthesis of antibiotic to hydrolysis of activated acyl donor abbreviated in literature as S/H) have been constructed by site directed mutagenesis. However, these variants of *E.coli* PA did not give the desired results. Organisms that have been found to produce penicillin acylases, for example *Acetobacter*, *Aeromonas*, *Alcaligenes*, *Aphanocladium, Alcaligerres faecalis, Xanthomonas sp., Providencia rettgeri, Xanthomonas citrii, Bacillus megaterium, E. coli,* of which *E. coli* PA is most commonly used.

As in most cases of *E.coli* based expression systems, the preproprotein is transported into periplasmic space where it undergoes post-translational modification to become a fully functional enzyme. This gives the possibility of using selective methods of cell breaking so that without too much of energy consumption, the respective enzyme can be released from the cells with relatively high specific purity. Breaking of cells can be done by classical mechanical methods like high pressure homogenization or disintegration in Beadmill, of which more often homogenization is followed at industrial level. Alternatively, organic solvents could be used to break the cells and to extract the product.

There are reports of using a combination of solvents to enhance cell lysis yield and purity of enzyme. De Leon et al in (2003), reported the use of various organic solvent to extract Penicillin G acylase from recombinant E. coli. (A. De Leon, B. Garcia, A. P. Barba de la Rosa, F Villaseñor, A.Estada, R.Lopez- Revilla, Process Biochemistry,39:301-305,2003) However, chemical lysis methodologies have to be custom developed depending on the microorganism and the enzyme sensitivity to the organic solvents.

Penicillin acylase can be used in thermodynamically controlled synthesis as well as kinetically controlled synthesis of β-lactam antibiotics.Penicillin acylase is used in the kinetically controlled synthesis of β-lactam antibiotics by direct condensation of the activated acyl donors with the respective β-lactam nucleus. These enzymatic processes, although seemingly competitive with traditional chemical synthesis, actually confront a major difficulty which determines the overall yield of an antibiotic, namely hydrolysis of the activated acyl donor as well as the antibiotic formed. (M.A.Wegman, U. Heinemann, A.Stloz, F.Van Rantwijk and R.A.Sheldon, Organic Process Res Dev. 4:318-322,2000,) The enzymatic synthesis of β-lactam antibiotics may work directly from the acyl donor, or from an ester or from amide derivative. (Kashe V. Enzyme Microbial Technology, 18 : 4-16,1986).

In the first case of thermodynamically controlled synthesis, the main issue is to shift the reaction equilibrium towards the products. Though presently this may not be successful, this would be an ideal and an elegant solution.

In the second case of kinetically controlled synthesis, an activated precursor of the acyl donor, either in ester form or in amide form of the D-acyl side chain of the antibiotic reacts with the β-lactam nucleus, releasing the antibiotic in addition to alcohol or ammonium plus, unreacted reactants and by products. Usually in kinetically controlled approach, the acyl donor is to be taken in molar excess in order to drive the reaction towards the synthesis, which makes the process unattractive. (Luciana R. B. Goncalves, Ruy Sousa, Jr. Roberto, Fernandez-Lafuente, Jose M.Guisan, Raquel L. C. Giordano, Roberto C. Giordano, Biotechnology and Bioengineering 8:6,622-631, 2002).

The major issues of this kinetically controlled enzymatic approach have been the yield, scalability and process economics due to excessive use of acyl donor. Many studies were aimed to improve this, which includes process optimization, addition of co-solvents or the use of high concentration of the substrates or improving the biocatalyst activity by immobilization.

Enzyme immobilization plays a very crucial role in stability and selectivity of the enzyme which in turn decides the future of the enzymatic process. Enzyme processes by and large compete with the well established chemical process and eventually get rejected if the process economics is not attractive. By and large, industrial enzyme immobilization has been quite successful since several years of which Penicillin G acylase is one of the successful enzymes used in pharmaceutical industry.

Usually for covalent type of immobilization, the target enzyme needs to be purified sufficiently which in turn increases the cost of production with increase in time. Also, specific traits of carrier material need to be studied to predict the suitability to particular protein as these carriers tend to alter the secondary, tertiary or quaternary structures of protein which subsequently hinder in their specific use.

This has also led to exploration of other possibilities of non-covalent immobilization. Entrapment and encapsulation have been most preferred methods of non-covalent immobilization.

Entrapment is particularly advantageous as it requires proteins in less purified form, even whole cells have been suitably entrapped in various carriers. The proteins in enzyme extract can be aggregated by addition of salts, organic or non-ionic polymers which induces protein to form super molecular forms and render them insoluble in aqueous solutions and the same can be cross linked with suitable agents to give cross linked enzyme aggregates. (A.M.van der Weilen, M.Ottens and A.J.J. Straathof et al, Straathof, A. J. J.Panke, S. and Schmid, A. Current Opin Biotechnol, 13:548-556 ,2002 and Cao, L, Van Langen, L. M. Van Rantwijk F et al, Enzyme Microbial Technology, 11: 665-670, 2001.Based on the above revelation and economic point of view, we have chosen entrapment as method of immobilization for recombinant PA from *Achromobacter sp.* CCM 4824.

The entrapment methods according to the invention for immobilization have either used whole cells or purified proteins which compromise on either the yield of expressed activity or the cost of enzyme purification. The enzyme used in the invention is partially-purified by precipitating the protein in saturated solution made by slow addition of precipitant like solid ammonium sulphate. (Protein Purification and Process Engineering Edited by Roger G Harrison, Marcel Dekker Inc. Publication,141-183,1993)

The object of enzyme immobilization includes high catalyst activity, high mechanical and operational stability, recyclability, high S/H ratios in case the enzyme is used in synthetic reactions and cost of manufacture.

Apart from above approaches, a more fundamental approach to enhance product yields is the use of new biocatalysts with improved kinetic properties, since it has been convincingly shown that kinetically controlled reactions are highly influenced by the kinetic parameters of the enzyme.

Consequently, current trends in research lead to the development of large number of new biocatalysts/ new variants of enzymes useful in chemical/ pharmaceutical industries. These new enzymes (bio catalysts) can be obtained by classical isolation of production microorganisms from nature, recombinant DNA technique from environmental gene pool or by an approach of directed evolution of enzymes resulting in a new family of penicillin acylases with improved synthetic potential.

US6403356 discloses mutant penicillin G acylases, for example from E. coli, which exhibit altered enzymatic activity. These penicillin G acylases are obtained by expression of a gene encoding said penicillin G acylase having an amino acid sequence which differs at least in one amino acid from the wild-type penicillin G acylase.

Thus the availability of a penicillin acylase with outstanding synthetic potential is a crucial factor for the development of enzymatic processes for β-lactam synthesis. One of the main kinetic parameters is the ratio of synthesis of antibiotic to hydrolysis of activated acyl donor (abbreviated in literature as S/H). This ratio is the key performance parameter of biocatalyst

Studies by Sheldon et al. (Sheldon, Van Rantwijk, Van Langen, Wegman, CAO and Janssen Synthesis of β-lactam antibiotics, Edited by Alle Bruggink, Kluwer Academic publication :126-129,2003) revealed that there is a great variation in hydrolytic activities as well as synthetic activities between different preparations of PA from E. coli.

| Enzyme | Carrier | Hydrolytic activity (U/g) |
|---|---|---|
| Poly-XDA-GA-PA | Poly-XDA-GA | 200 |
| Assemblase 7500 | Gelatin-chitosan | 260 |
| PGA 450 | Special polymer | 210 |
| PcA | Eupergit C | 310 |

Of the above examples,. Poly XDA-GA-PA (microbeads from 1,4-di(amino methyl) benzene (p-xylylene diamine, by glutaraldehyde activation) proved to be an efficient catalyst for β-lactam synthesis. In case of Ampicillin synthesis, the S/H ratio with this enzyme preparation was 2.0 at 85% conversion, which was comparable with free enzyme and better than Assemblase (S/H 1.22 at 80% conversion). In case of Amoxicillin synthesis, the same enzyme performed well with S/H ratio of 4.5 upto 75 % conversion which is slightly higher than that with free Penicillin acylase.

Penicillin acylase (PA; E.C. 3.5.1.11, penicillin amidohydrolase) from *Achromobacter* sp. CCM 4824 (originally *Comanionas testosteroni* CCM 4824; Plhá 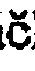 ková et al. (K. Plhá 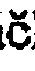 ková et al. Applied Microbial Technology, 32: 507-516, 2001), 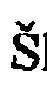 krob et al., Enzyme Microbial Technology 32: 738-744, 2003) exhibits better traits as regards the enzymatic syntheses of Ampicillin, Amoxicillin, Cephalexin and Cefadroxil when compared to PA from outer sources.

Kyslik et al. (Czech patent application PV 2007-82) on further work cloned and expressed the above Penicillin acylase from *Achromobacter* CCM4824 in recombinant strain E. *coli* BL21(pKX1P1) CCM7394 on a plasmid vector. The method of cloning and expression of this gene and its fermentation conditions are described in Czech patent application (PV-2007-82) as well in Indian PCT application PCT/IN07/00193 (published as WO 2008/093351). The present invention involves the application of Penicillin acylase as derived above in industrial synthesis of β-lactam antibiotics.

Thus the present invention predominantly involves in extraction of penicillin acylase from *E. coli* BL21(pKX1P1) and subsequent synthesis of β-lactam antibiotics by using Penicillin acylase, a new biocatalyst, to obtain final product with improved yields.

### Objects of the invention:

The main object of the invention is to provide Penicillin Acylase (PA) from *Achromobacter sp.* CCM 4824 expressed in recombinant strain *E. coli BL21* bearing the recombinant plasmid pKX1P1(CCM 7394), as biocatalyst.

Another object of the invention encompasses a process of cell lysis, enzyme isolation, preparation of biocatalyst by enzyme stabilization and designing parameters for efficient synthesis of β-lactam antibiotics.

Yet another object of the invention provides industrial synthesis of β-lactam antibiotics using the immobilized enzyme.

### Summary of the invention

The present invention relates to Penicillin Acylase (PA) from *Achromobacter* sp. CCM 4824 as isolate from the recombinant strain E. *coli* BL21 CCM7394 bearing the recombinant plasmid pKX1P1 and processing of PA into biocatalyst useful for the industrial synthesis of antibiotics.

Accordingly the process of the invention for synthesis of semi-synthetic penicillin or cephalosporin antibiotic using Penicillin Acylase biocatalyst from *Achromobacter* sp CCM 4824 expressed in *Escherichia coli BL21* CCM 7394 comprises:
a. chemically extracting Penicillin Acylase using solvent and chemical detergents or metal chelators for cell permeabilization;
b. purifying the soluble enzyme by fractional precipitation and chromatography;
c. immobilizing the said enzyme using acrylamide and N,N'-Methylenebisacrylamide in a ratio ranging from 15:1 to 20:1 at a temperature of-2 to 2°C;
d. stabilizing the immobilized enzyme by crosslinking with glutaraldehyde to obtain coarse particles with particle size ranging from 100 to 300 microns, preferably between 150 to 250 microns and
e. enzymatically acylating the nucleophile with activated acyl donor using the above immobilized Pencillin Acylase enzyme to obtain the β-lactam antibiotic with high yield and purity.

The solvent used in above extraction is selected from toluene, ethylacetate, chloroform and butylacetate along with chemical detergents or metal chelators selected from the group comprising EDTA, cetrimide, urea and sodium lauryl siulphate. The extraction process utilizes the effective combination of chloroform 1 to 3% w/v and sodium lauryl sulphate 0.1 to 0.2% w/v.

The extraction is carried out at a temperature of 15 to 35°C, preferably at 28 to 30°C for 6 to 12 hrs by maintaining the pH in the range of 5.5 to 9.0, preferably between 6.5 to 7.5 to obtain soluble enzyme.

The purification of the enzyme is carried out either by treating with ammonium sulphate or by using ion-exchange chromatography.

The enzyme immobilization comprises enzyme in aggregated form which is pre-treated with ammonium sulphate at an amount corresponding to 30 to 70% of saturation. The aggregates are achieved using acrylamimide and methelene bis-acrylamide in a ratio ranging from 15:1 to 20: 1. The enzyme immobilization process is carried out in sodium or potassium phosphate buffer by crosslinking with glutaraldehyde in a concentration of 2mM to 8mM, preferably between 4mM to 6mM.

The immobilization of the enzyme further comprising a step of secondary crosslinking with glutaraldehyde at a concentration ranging from 0.3 to 0.5%(w/v).

The process of enzymatic acylation for the preparation of semi-synthetic penicillin or cephalosporin antibiotic comprising reacting a mixture containing the nucleophile with activated acyl donor along with the above immobilized Pencillin Acylase enzyme at a pH ranging between 5.2 to 7.2. The pH of the reaction mixture is adjusted by the addition of an acid, preferably conc. HCl.

The acyl donar is selected from D-p-hydroxyphenylglycine methyl ester or amide or D-phenylglycine methyl ester or amide and the nucleophile is selected from 6-Aminopenicillianic acid or 7-Amino Deacetoxycephalosporanic acid.

The synthesis of antibiotic is further comprising a step of removing a portion of semi-synthetic penicillin or cephalosporin antibiotic from the reaction mixture by lowering the pH of the said reaction mixture.

The semi-synthetic penicillin or cephalosporin antibiotic, Amoxicillin is synthesized by acylation of 6-amino penicillanic acid with D-Hydroxy phenyl glycine methyl ester, in a molar ratio ranging from 1.2 to 4.5, more preferably between 1.5-1.8

The semi-synthetic penicillin or cephalosporin antibiotic, Ampicillin is prepared by acylation of 6-amino penicillanic acid with D-phenyl glycine methyl ester, in a molar ratio ranging from 1.2 to 4.5, more preferably between 1.3 to 1.7.

The semi-synthetic penicillin or cephalosporin antibiotic, Cephalexin is prepared by acylation of 7-Amino deacetoxy cephalosporanic acid with D-phenyl glycine methyl ester, in a molar ratio ranging 1.2 to 4.5, more preferably between 1.5 to 1.7.

The process of the invention further discloses cultivation of genetically modified *E. coli* BL21 CCM 7394 containing PA from *Achromobacter* sp CCM 4824 in fed-batch mode and this process predominantly includes, cell lysis, enzyme isolation, preparation of biocatalyst by enzyme stabilization and designing parameters for efficient synthesis of Amoxicillin, Ampicillin, Cefadroxil and Cephalexin using immobilized recombinant Penicillin Acylase as biocatalyst isolated from *Achromobacter sp* CCM 4824 as biocatalyst

The immobilization process of Penicillin acylase was also suitably designed to enhance the synthetic ability of the immobilized enzyme and retention of increased activity for longer period to ensure stability of the biocatalyst.

### Description of drawings:

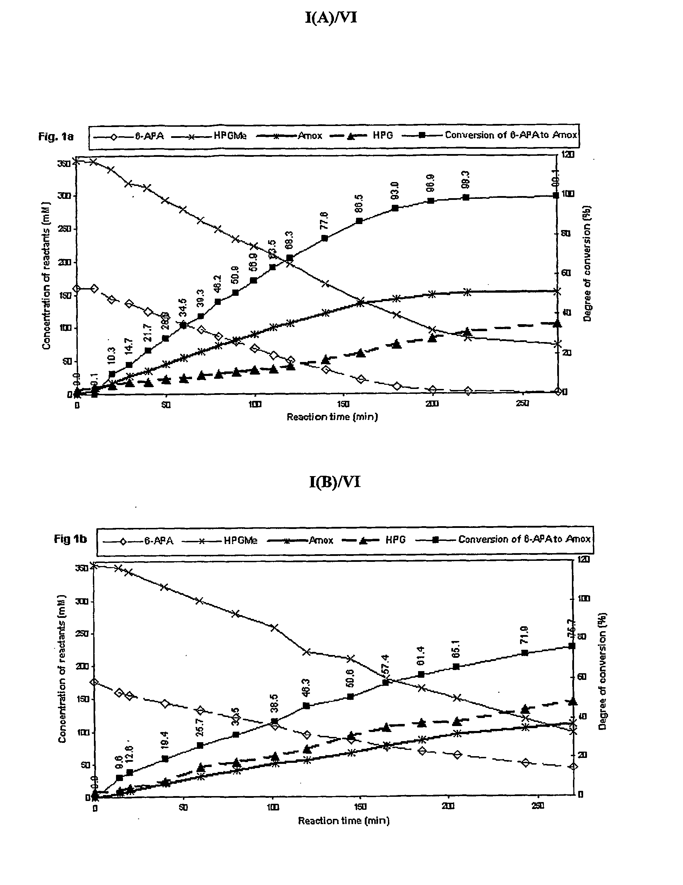

Fig 1.a.(IA/VI) shows the course of conversion of synthesis of Amoxicillin trihydrate (AMOX) from 6-APA (nucleophile) and HPGMeHCl (acyl donor) using FERMASE NA^{™} 150.(Example 8)
Fig 1.b.(IB/VI) shows the course of conversion of synthesis of Amoxicillin trihydrate (AMOX) from 6-APA (nucleophile) and HPGMeHCl (acyl donor) using and FERMASE PA^{™} 1500.(Example 8).
Fig 2.a(IIA/VI). shows the course of the conversion in the synthesis of AMOX from 6-APA and HPGMeHCl using FERMASE NA^{™} 150 and AMOX purification.(Example 9)

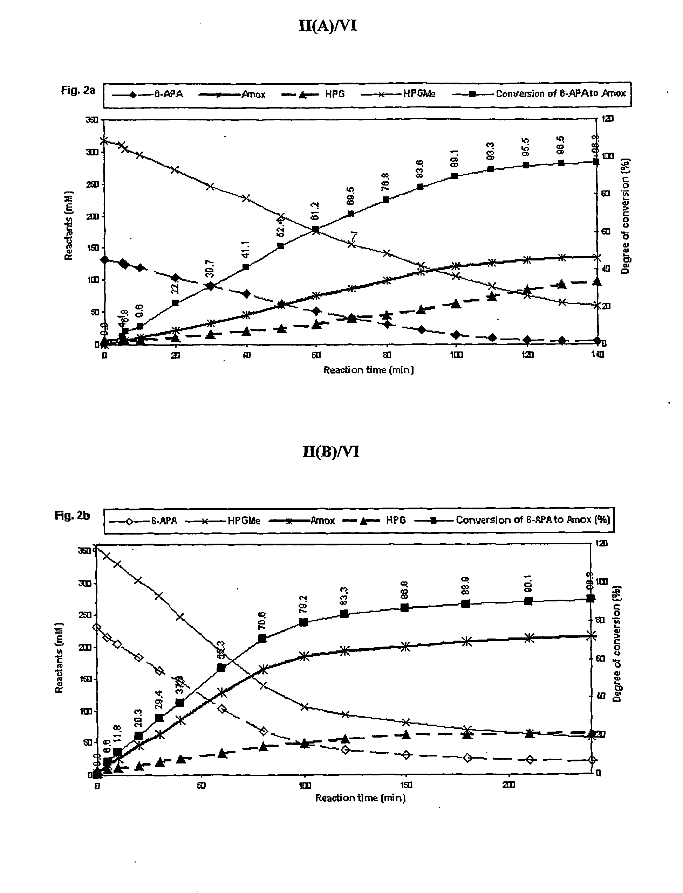

Fig 2.b.(IIB/VI) shows the course of the conversion in pH-controlled synthesis of AMOX from 6-APA and HPGMeHCl using FERMASE NA 150 .Acyl donor/nucleophile ratio 1.49 (Example 10)

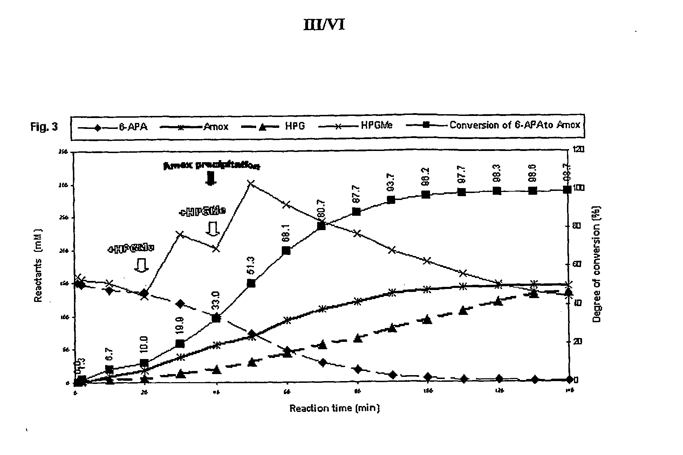

Fig 3.(III/VI) shows the time course of the conversion in the synthesis of AMOX from 6-APA) and HPGMeHCl) using FERMASE NA^{™} 150 and AMOX purification. (Example 11)

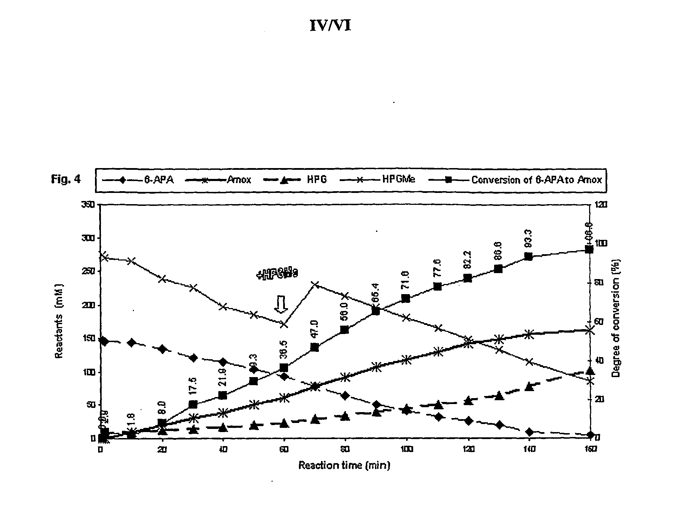

Fig 4(IV/VI) shows the time course of the conversion in the synthesis of AMOX from 6-APA and HPGMeHCl using FERMASE NA ^{™}150 - batch reaction with postponed acyl donor addition(Example 13)

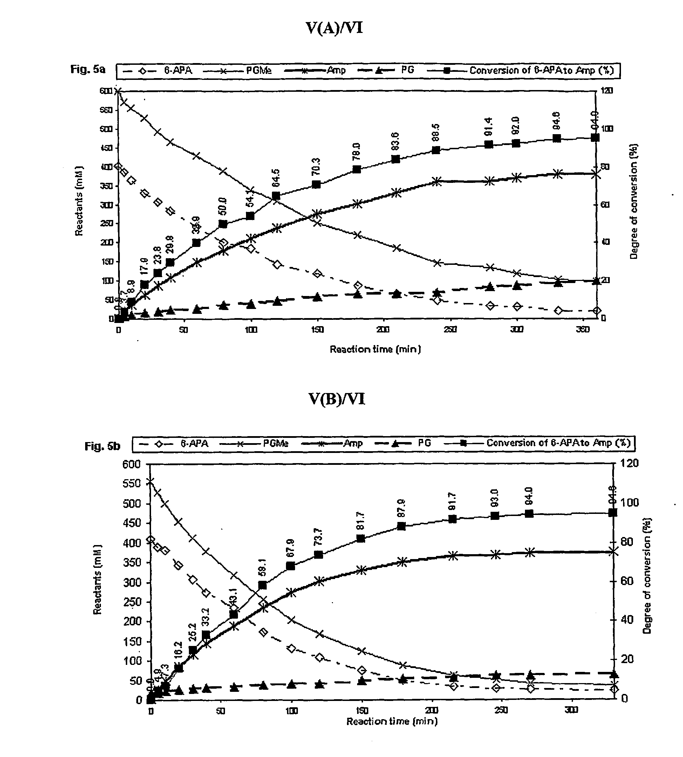

Fig 5.a.(VA/VI) shows the course of the conversion in the synthesis of Ampicillin trihydrate (AMP) from 6-APA and PGMeHCl using FERMASE NA 150.Acyl donor/nucleophile ratio 1.5(Example 19).
Fig 5.b(VB/VI) shows the course of the conversion in the Synthesis of AMP from 6-APA and PGMeHCl using FERMASE NA 150 Acyl donor/nucleophile ratio 1.35 - Batch reaction at pH 5.9, 20°C (Example 20).

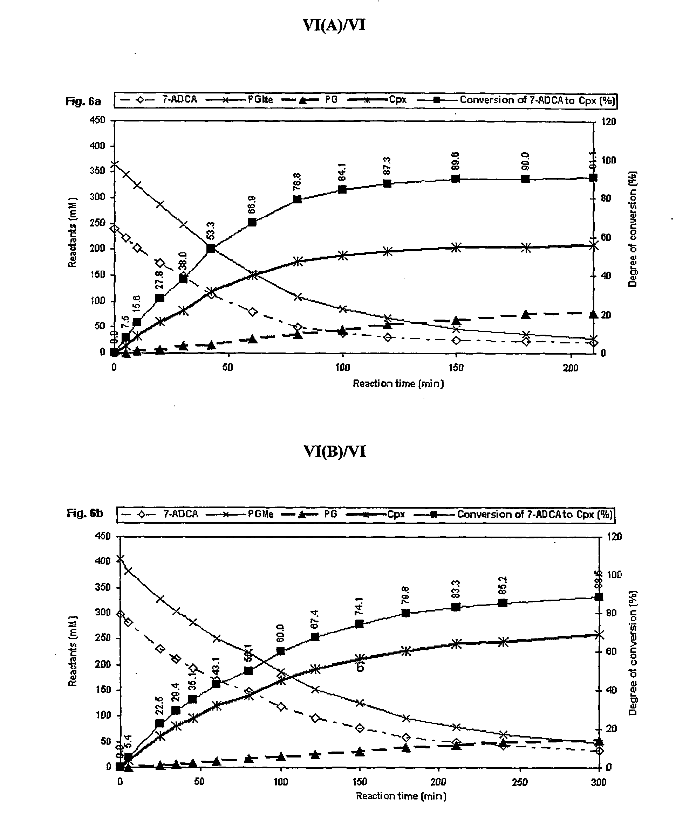

Fig 6.a.(VIA/VI) shows the course of the conversion in the Synthesis of cephalexin monohydrate (CEX) from 7-ADCA and PGMeHCl using FERMASE NA 150.Acyl donor/nucleophile ratio 1.5 - Batch reaction at pH 6.3, 25°C.(Example 21).
Fig 6.b.(VIB/VI) shows the course of the conversion in the Synthesis of CEX from 7-ADCA and PGMeHCl using FERMASE NA 150_acyl donor/nucleophile ratio 1.34 - Batch reaction at pH 6.3, 25 °C(Example 22).

### Detail description of the invention:

The present invention is related to field of enzymatic approach for the preparation of commercially important semi-synthetic penicillins and cephalosporins (e.g Ampicillin, Amoxicillin, Cephalexin, Cefazolin, Cefadroxil)

The process of the invention involves the isolation of recombinant Penicillin Acylase from *Achromobacter sp. CCM 4824* expressed in genetically modified E. *coli* BL21 CCM 7394 cells and this process predominantly includes, cell lysis, enzyme isolation, preparation of biocatalyst by enzyme stabilization and designing parameters for efficient synthesis of Amoxicillin, Ampicillin and Cephalexin using immobilized recombinant Penicillin Acylase as biocatalyst isolated from *E. coli* CCM 7394. The method of cultivation of the recombinant strain CCM 7394 is described in Czech patent PV-2007-82.

According to the invention semi-synthetic penicillin or cephalosporin antibiotics are synthesized from the reaction mixture consisting of activated acyl-donor (D-p-hydroxyphenylglycine methyl ester or amide for Amoxicillin and Cephadroxil; D-phenylglycine methyl ester or amide for Ampicillin and Cephalexin) with nucleophile (6-APA or 7-ADCA) catalyzed by recombinant penicillin Acylase derived from *Achromobacter sp.* CCM 4824

The disclosure provides method of cell lysis of *E.coli BL21* CCM 7394 cells containing Penicillin Acylase from *Achromobacter sp.CCM* 4824,using various methods such as high pressure homogenizer; mild chemical extraction using various solvents such as ethyl acetate, tolune; butyl acetate and chloroform. The degree of lysis using different solvents is measured by drawing the samples periodically from the reaction medium. The degree of lysis caused due to the solvent is measured by assaying A^{H}_{PenG} in supernatant samples and comparing with initial activity of cell suspension (%cell lysis). The method of cultivation of the recombinant strain CCM 7394 is described in Czech patent PV-2007-82.

The experimental results have proved that chloroform is the most suitable solvent with a yield of 60% and hence further experiments were done to optimize the enzyme yield with high SA^{H}_{PenG}. The results are shown in Table 1

By following the above procedure, various concentrations of chloroform were added to initiate the chemical lysis and subsequent extraction of the enzyme is carried out. At 2% v/v chloroform concentration, the cell lysis observed is 80% when compared with other concentrations. The results were given in Table2

Further, to improve the degree of cell lysis, different supplements such as chemical detergents and metal chelators selected from EDTA (Ethylene diamine tetraacetic acid), cetrimide, urea and sodium lauryl sulphate are added to the chloroform and observed that the use of sodium laurylsulphate as a supplement enhances the degree of lysis. The results are shown in Table 3. This reaction can be conveniently carried out at a temperature range of 15 to 35°C.

Further, the lysed biomass is stored for 12 hrs at 5°C to allow the separation of protein from the ruptured cells. The temperature of the suspension was raised to 28 to 30°C and diluted with distilled water; and the pH is adjusted to 5.0 using 2M acetic acid. The coagulation of acid protein from the suspension is done by adding polyethylene imine at 40°C for 1 hr. The flocculated material is separated from the solution by centrifugation. The pH of the clear supernatant liquid is adjusted to 7.5 with 4N sodium hydroxide and the obtained solution is designated as soluble enzyme (SE).

Part of the above SE solution is stirred at 5°C and pH is adjusted to 7.5. To this, solid ammonium sulphate, 390 grams per liter, equivalent to saturation of 60% (w/v) was slowly added over a period of 20minutes.On further stirring at same temperature for 60minutes. The saturated solution is refrigerated for 12 hrs and the precipitated enzyme is isolated at 5°C. The precipitate contained A^{H}_{PenG} of 700 units per gram and specific activity of 2 units per mg protein.
The disclosure describes preparation of enzyme paste by addition of 283.6 grams per liter of solid ammonium sulphate to the pre cooled SE (0-1°C) in the course of 30minutes. Precipitation proceeded for 2hrs followed by centrifugation to obtain the enzyme paste. The yield of enzyme was 74%.The enzyme pastes obtained in the above were used in immobilization into polyacrylamide gel or further for purification of PA as described below.

In an embodiment, the process of the invention provides a purification step of penicillin acylase using ion-exchange chromatography method. Chromatography on CATEX with functional carboxy groups, a weak cation-exchanger, was applied to prepare purified PA from enzyme paste suitable for immobilization on macroporous polyacrylate resins such as Sepabeads and DILBEADS ^{™}. The PA is selectively bound to the ion-exchanger and the bulk protein with lower isoelectric point went through the column. Bound PA was recovered from the column with a gradient of Potassium Chloride. The solution of purified enzyme contains two isoforms of identical SA^{H}_{penG} present in weight ratio 1: 2.7. Total yield of activity after this purification is equal to 45%.

The immobilization process of Penicillin acylase was also suitably designed to enhance the synthetic ability of the immobilized enzyme and retention of increased activity for longer period to ensure better stability of the biocatalyst. The immobilization process involves a method of enzyme entrapment. Polyacrylamide based polymers are most commonly used for enzyme entrapment, because their degree of similarity to those of free enzymes The immobilization yields achieved are nearly 90% in terms of protein binding and almost 60-70% in terms of expressed activity. The limitation in use of poly acrylamide is the leakage of the enzyme from the matrix due to non-reactive nature of the polymer, physical stability and also diffusion problem encountered to a large substrate molecule. Both these limitations have been overcome by use of optimized degree of cross linking. Of all the other cross linking agents, Glutaraldehyde, a bi-functional cross linker is agent of choice for Polyacrylamide based gel entrapment. (Protein Immobilisation-Fundamentals and Application, Edited by Richard Taylor, Marcel Dekker Inc Publication, 40:1991)

The main criteria of this optimization are to retain the synthetic enzyme activity though certain degree of inactivation is unavoidable and also to impart mechanical strength to the polymerized gel to limit leakage of enzyme from polymer. The gel entrapped enzyme is further recrosslinked to ensure better stability of the biocatalyst as a final product.

The method is more suitably adapted to have considerable amount of nonspecific proteins in precipitation to aid better cross linking with glutaraldehyde. This also helps to accelerate the polymerization process as well. The use of enzyme with low specific activity makes the process economical and with higher yield in terms of product and activity.

100 grams of enzyme paste obtained as above is diluted with sodium phosphate buffer to obtain a homogenized solution. 14.5 ml from the stock solution containing Acrylamide monomer solution (10% w/v) and N',N'-methylenebisacrylamide (0.5%w/v) were added to above stirred enzyme solution at 5°C and further continued stirring for 5minutes. To this reaction mass, 5ml of 25% (w/v) solution of glutaraldehyde was added and allowed to stir for exactly 8minutes. Then 0.5% (v/v) solution of TEMED and 1.0 % (w/v) freshly prepared solution of ammonium persulfate were added to the solution. Polymerization is initiated within 2minutes.

The amount of ammonium persulphate and TEMED used for the purpose of complete polymerization is studied and the results were described in Table 6. It has been confirmed that the ratio 1: 0.6 of ammonium persulphate and TEMED is the best one to achieve the complete polymerization.

A thick, opaque polymerizing mass obtained which was poured into pre-chilled trays and allowed to fully polymerize and incubated for 30minutes The formed gel aggregate was cut and sieved through #30 mesh (500 micron) sieve. The resulting gel aggregate is washed through # 50 mesh (300 micron) with water to remove finer particles, traces of un-reacted monomer and other reagents.

Further washing of the entrapped enzyme is done with 1.2 M Sodium Chloride to remove traces of unbound protein.

To enhance crosslinking, the aggregate is stirred in 0.5% (v/v) solution of Glutaraldehyde at 25°C for 30minutes. After re-crosslinking, traces of Glutaraldehye are removed by washing with 5 volumes of water.

Finally the catalyst is vacuum filtered to remove excess water and stored in 50mM Sodium Phosphate buffer, pH 7.8 containing 500 ppm, Ethylparaben. The % dry weight of the final product ranges between 12-15%, with particle size distribution of 98% particles between 100-300 microns.

The yield in terms of weight ranged from 88 to 92 %. The yield of expressed activity ( A^{H}_{PenG} = 110 U/g ww; A^{H}_{PenG}= 750 U/g dw) is equivalent to 47% with respect to the activity taken for immobilization.

The activity is determined by alkalimetric titration using 25mM Penicillin G, 50mM sodium phosphate buffer pH 8.0, at 37°C. The amoxicillin synthetic activity (A^{S}_{Amox} =150U/g dw) is determined at reaction conditions using 40mmoles of 6-APA and 60 mmoles of HPGMeHCl, at pH 6.3 and 40°C. The reaction is carried out for 30minutes, followed by HPLC analysis for activity determination.

The above process is repeated by using variable concentrations of Acrylamide and N',N'-methylene bisacrylamide to ascertain the best workable ratio of the same for enzyme entrapment. The best workable concentration ratio of Acrylamide to N',N'-methylene bisacrylamide is found to be 19:1 with a resultant yield 92% of enzyme activity. The experimental results to this extent are shown in Table 5.

The above experiment is further continued with variable concentrations of glutaraldehyde chosen from 1 to 5 mille mole per milligram of protein used for cross-linking. The results obtained are indicated that the increase in concentration of glutaraldehyde increases the strength of the biocatalyst formed but yield of activity is inversely related to concentration of glutaraldehyde. The results are shown in Table 6.

The disclosure describes the evaluation of hydrolytic activity and polyacrylamide gel aggregate (FERMASE NA ^{™} 150) and (FERMASE NA ^{™} 1500) at 25°C and at 37°C and observed that the activity of the catalyst is found nearly two fold at higher temperature.

The disclosure is also directed to the synthesis of Amoxicillin trihydrate from 6-APA (nucleophile) and HPGMeHCl(acyl donor) using FERMASE NA ^{™} 150 and FERMASE NA^{™} 1500.

In the process of the invention, acyl-donor is used in molar excess to shift the threshold of the reaction towards the product, an antibiotic. Both reactants (acyl donor, nucleophile) may be added into the batch-type reaction at once in the beginning of the reaction which results in formation of thick suspension due to low solubility of acyl donor, or acyl donor may be added into the reaction in a step-wise mode to prevent formation of the suspension. In the later case, the reaction solution is clear until the less-soluble product (antibiotic) is formed, the initial rate of the hydrolysis of the acyl donor is lower and the process can be operated in continuous mode. Both ways of addition of acyl donor may be combined.

There is tendency of pH to decrease in the course of reaction so that it is necessary to adjust the pH of reaction mass. The specific final pH of 6.3 is necessary to achieve the maximum conversion of 6-APA into Amoxicillin trihydrate. The pH is adjusted using phosphoric acid or sodium hydroxide or ammonia.

Similar experiments were carried out for the synthesis of Amoxicillin from 6-APA (nucleophile) and HPGMeHCl (acyl donor) using FERMASE PA ^{™} 1500 at controlled pH conditions.

The reaction is terminated based on the % conversion of 6-APA with concomitant increase in Amoxicillin. The enzyme is removed from the reaction mass and washed with water thoroughly for further use.

In similar manner, the other semi-synthetic penicillin or cephalosporin antibiotics are synthesised using recombinant PA as biocatalyst obtained from *Achromobacter* sp. CCM 4824. The condensation of 6-APA(nucleophile) and PGMeHCl(as acyl donor) for the synthesis of Ampicillin; 7-ADCA (nucleophile) and UPGMeHClester or amide (acyl donor) for the synthesis of cefadroxil; 7-ADCA (nucleophile) and PGMeHCl ester or amide (acyl donor) for the synthesis of Cephalexin.

### Abbreviations:

- A^{H}_{PenG}: activity of hydrolysis of PenG
- SA^{H}_{PenG}: activity expressed per mg protein
- SE: soluble enzyme prepared according to Example 5
- PA: penicillin acylase
- PGA: penicillin G acylase
- rPA_{As}: recombinant PA from *Achromobacter* sp.
- rPGA_{Ec}: recombinant PGA from *Escherichia coli*
- HPGMeHCl: D-p-hydroxyphenylglycine methyl ester Hydrochloride
- PGMeHCl: D-phenylglycine methyl ester Hydrochloride
- HPG: D-Hydroxy phenyl glycine
- PG: D-Phenyl Glycine
- AAD: activated acyl donor
- Wn: % Conversion with respect to initial nucleophile
- AMOX: amoxicillin trihydrate
- AMP: ampicillin trihydrate
- CEX: cephalexin monohydrate
- A^{H}_{AAD}: activity of hydrolysis of activated acyl donor
- A^{S}_{Amox}: activity of AMOX synthesis
- A^{S}_{Amp}: activity of AMP synthesis
- A^{s}_{Cex}: activity of CEX synthesis
- S/Hᵢₙᵢₜ: ratio of A^{S}_{Amox}, A^{S}_{Amp} or A^{S}_{Cex} / A^{H}_{AAD}
- RM: reaction mixture
- cdw: cell dry weight
- dw: dry weight
- ww: wet weight
- TEMED :: N',N,'N,'N'-tetramethylethylenediamine
- Cell paste: : E.coli BL 21 cells having PA from Achromobacter sp
- FERMASE PA ^{™}1500 :: Polyacrylamide gel entrapped rPGA_{Ec}
- FERMASE NA ^{™} 150:: Polyacrylamide gel entrapped rPA_{As}
- RPM: Revolution per minute
- U: Activity Units (U): Defined as the amount of enzyme catalyzing the formation of 1 micromole of 6-APA at 37 °C per minute pH 8. at 53mM Penicillin G K potassium salt, in the milleu of 50 mM sodium phosphate buffer within the range of zero order kinetics.

The following examples, which include preferred embodiments, will serve to illustrate the practice of this invention, it being understood that the particulars shown are by way of example and for purpose of illustrative discussion of preferred embodiments of the invention.

### Examples:

### Example 1

### Cell lysis by high pressure homogeniser

*E.coli BL 21* CCM 7394 cells with the activity of PA from *Achromobacter sp*.CCM 4824 were harvested from the culture broth by centrifugation in a form of cell paste (Sharples centrifuge) or in a form of cell suspension (Alfa-Laval continuous separator). The harvested biomass was suspended in 0.02M sodium phosphate to get about 5 % (cdw/v) suspension pH of which was adjusted to 7.0 and maintained at this value with 20%w/v sodium hydroxide. Cell suspension (2350 ml, 4.9% cdw/v, A^{H}_{PenG} of 86.5 U/ml) cooled to 8-13°C was subjected to disintegration (homogenizer Manton-Gaulin, pressure of 40-50 MPa, three consecutive cycles). After each cycle, the homogenate was cooled down to 8-13°C and pH was adjusted to 7.0. The cell debris and a fraction of ballast proteins were removed by thermocoagulation step consisting in: pH adjustment to 5.0 with 50% acetic acid, addition of the flocculant Sedipur CL 930 (2-4g/L) and maintaining the temperature at 40°C for 1 hour. After fast cooling of the homogenate to 8-13°C, the homogenate was diluted with 700 ml H₂O, pH was adjusted to 8.0 (20% sodium hydroxide solution) and the mixture was centrifuged (Beckman centrifuge, angle rotor,4 X 350 ml, 10,000 rpm) for 20 min. pH of the clear enzyme solution (2580 ml, 8.7 mg protein/ml, A^{H}_{PenG} of 64.8 U/ml) was adjusted to 7.0 and enzyme solution was frozen at -16 °C. The yield of the enzyme (S A^{H}_{PenG} = 8.4 U/mg protein) was 82 % (the activity of the cell suspension before disintegration equals 100%).

### Example 2

### A. Enzyme extraction by mild chemical extraction

In another embodiment of invention, cell suspension (1050 ml, 5% cdw/v, A^{H}_{PenG} of 10.2U/ml) was suspended in 0.1M sodium phosphate buffer (pH 7.5) at 20°C. A mild chemical lysis was carried out in three-necked glass reactor equipped with pH probe, temperature probe and variable agitator. After adjusting the pH to 7.0 with diluted ammonium hydroxide solution, the temperature was brought to 28°C. To this suspended biomass, 1% (v/v) of solvents like Toluene, Ethyl acetate, Chloroform, Butyl acetate was added to initiate cell lysis The reaction was monitored for change in pH at constant temperature. Samples were withdrawn periodically to estimate degree of cell lysis. The reaction was continued for 2hrs at pH 7.0 and 28°C. The degree of lysis caused due to the solvent was measured by assaying A^{H}_{PenG} in supernatant of samples and comparing with initial activity of cell suspension (% cell lysis, Table 1).

**Table 1:**

| Experiment | Solvents | % Cell lysis |
|---|---|---|
| 1 | Toluene | 7.4 |
| 2 | Ethyl acetate | 25 |
| 3 | Chloroform | 60 |
| 4 | Butyl acetate | 33 |

Results indicated that Chloroform gave the best results with yield of 60% and hence further experiments were done to optimize the enzyme yield with high SA^{H}_{PenG}.

### B. Enzyme extraction by mild chemical extraction with chloroform

By following the same procedure described above, various concentrations of Chloroform were added to initiate the chemical lysis and subsequent extraction of enzyme.

**Table 2:**

| Examples | Chloroform concentration | % Cell lysis |
|---|---|---|
| | (in %, v/v) | |
| A | 1 | 62 |
| B | 2 | 80 |
| C | 2.5 | 74 |
| D | 3 | 45 |

It was evident that Chloroform used at concentration of 2% (v/v) was effective to achieve maximum degree of lysis as shown in the above Table 2. In the case of higher concentrations of chloroform the lower yield is due to denaturation of the enzyme by solvents.

### C. Enzyme extraction by mixture of chloroform and different supplements

By following the same procedure described above, various concentrations of chemical detergents and metal chelators were added along with Chloroform to enhance the chemical lysis and enzyme yield. Substances like EDTA (Ethylene diamine tetraacetic acid), Cetrimide and Urea are known to help in cell lysis, either individually or in combination. The addition of Sodium lauryl sulphate enhances the degree of lysis. The results were shown below in Table 3.

**Table 3**

| Examples | Chloroform concentration | supplement | % Cell lysis |
|---|---|---|---|
| | (in %, v/v) | (0.2% w/v) | |
| A | 2 | EDTA | 85 |
| B | 2 | Cetrimide | 45 |
| C | 2 | Urea | 33 |
| D | 2 | Sodium lauryl sulphate | 92 |

### Example 3

### Preparation of enzyme concentrate

350gm of cell paste prepared as in Example 1 was suspended in distilled water to final concentration of 5% (cdw/v) at 20 - 25°C. The pH of the cell suspension was adjusted to 7.0 and ice cold 2% (v/v) Chloroform and 0.2% (w/v) Sodium lauryl sulfate was added to initiate lysis. Samples were withdrawn periodically during the total period of 5 hrs. The lysed biomass was further stored at 5°C for 12hrs before further processing to allow separation of protein from the ruptured cells. The temperature of the suspension was raised to 28- 30°C and diluted two fold with distilled water. The pH was adjusted to 5.0 with 2M acetic acid. Coagulation of acid protein from the suspension was done by adding 1% (w/v) of Polyethylene imine at 40°C for one hour. The flocculated material was separated from the solution by centrifugation at 7000rpm. The pH of clear supernatant was adjusted to 7.5 with 4N sodium hydroxide solution and obtained solution was designated as soluble enzyme (SE). The enzyme yield with respect to initial activity of cell paste was 90%. Table 4 shows the yield of PA activity in the course of preparation. This soluble enzyme (SE) was used further for preparation of catalyst.

**Table 4:**

| Time of extraction | Total A^{H}_{PenG} | yield of activity |
|---|---|---|
| | (U) | (%) |
| cell paste suspension | 5670 | 100 |
| 1hr | 4535 | 80 |
| 2hr | 5330 | 94 |
| 3hr | 5500 | 97 |
| 5hr | 6297 | 111 |
| 12hr | 6237 | 110 |
| Soluble enzyme | 5106 | 90 |

### Example 4

### Purification of enzyme paste by ammonium sulphate treatment

### Method A

Enzyme solution prepared as in Example 1 (2580 ml, 8.7 mg protein/ml, A^{H}_{PenG} of 64,8 U/ml, pH 7.0) was further purified by precipitation with ammonium sulfate. Solid ammonium sulfate (283.6 g/L) was added to the pre-cold enzyme solution (0-1°C) in the course of 30 minutes under cooling. Precipitation proceeded for 2 hrs and the precipitate was removed by centrifugation. The enzyme paste was used for immobilization into polyacrylamide gel or for further purification of PA. The yield of the enzyme was 74 % (the activity of the cell suspension before disintegration equals 100%).

### Method B

Part of SE prepared in Example 3 was stirred at 5°C and pH was adjusted to 7.5. Solid Ammonium sulphate, 390 grams per liter, equivalent to a saturation of 60% (w/v), was slowly added over a period of 20mins.

On further stirring 5°C for 60minutes; the saturated enzyme solution was refrigerated for 12hrs. Enzyme precipitate was separated by centrifugation (Remi Centrifuge) at 7000 rpm at 5°C for 30 minutes. The precipitate contained A^{H}_{PenG} of 700 units per gram and specific activity of 2 units per mg protein.

### Method C

### Purification of PA by ion-exchange chromatography:

Chromatography on CATEX with functional carboxy groups, a weak cation-exchanger, was applied to prepare purified PA from enzyme paste suitable for immobilization on macroporous polyacrylate resins such as Sepabeads and DILBEADS^{™}. As an weak cation-exchanger, Fractogel COO⁻ (Merck) equilibrated with 0.02M sodium phosphate buffer (pH 7.0) was used. The paste (prepared in Example 6) was dissolved in 0.1M sodium phosphate buffer (pH 7.0, 400 ml) and thoroughly dialyzed against 0.02M sodium phosphate buffer (pH 7.0) so that the conductivity of the sample and the buffer is the same. The solution was centrifuged and the pellet was discarded. A^{H}_{PenG} in supernatant equalled 259 U/ml and SA^{H}_{PenG} = 16.8 U/mg protein. The solution was applied onto the column, the PA was selectively bound to the ion-exchanger and the bulk protein with lower isoelectric point went through the column. Bound PA was recovered from the column with gradient of potassium chloride (0.0 - 0.14 M potassium chloride in 0.02 M sodium phosphate buffer, pH 7.0). To isolate PA from the 0.4 l of dialyzed solution, a glasscolumn with bedvolume of 1 l was used. The solution of purified enzyme (volume of 1.25 l, A^{H}_{PenG} of 43.4 U/ml) contained two isoforms of identical SA^{H}_{PenG} (38.1 and 42.6 U/mg protein) present in weight ratio 1: 2.7. Total yield of activity after this step of purification equalled 45%.

### Example 5

### Preparation of Polyacrylamide gel aggregates:- FERMASE NA^{™} 150

### Method A

The enzyme paste obtained from Example 3A was diluted with 0.3M sodium phosphate buffer (pH 7.5) to get 75ml of solution of PA (A^{H}_{PenG} of 250 U/ml). The solution was kept in stirring at 5°C. 14.5ml of 10% (w/v) solution of Acrylamide monomer and 0.5% (w/v) solution of N',N'-methylenebisacrylamide was added to enzyme solution and stirred for 5 minutes. 5ml of 25% (v/v) Glutaraldehyde was added and allowed to stir for exactly 8 minutes. Then, 0.5% (v/v) solution of TEMED and 1.0 % (w/v) freshly prepared solution of Ammonium persulfate were added to the solution. Polymerization was initiated within 2 minutes.

At this stage, a thick, opaque polymerizing mass was poured into pre-chilled trays and allowed to fully polymerize and incubated for 30 min. The formed gel aggregate is cut and sieved through #30 mesh (500 micron) sieve. The resulting gel aggregate is washed through # 50 mesh (300 micron) with water to remove finer particles, traces of un-reacted monomer and other reagents.

Further washing of the entrapped enzyme was done with 1.2 M sodium chloride prepared in 5 times of the volume to remove traces of unbound protein.

To enhance crosslinking, the aggregate was stirred in 0.5% (v/v) solution of Glutaraldehyde at 25°C for 30 minutes. After re-crosslinking, traces of Glutaraldehyde were removed by washing with 5 volumes of water.

Finally the catalyst was vacuum filtered to remove excess water and stored in 50mM Sodium Phosphate buffer pH 7.8, containing 500 ppm of Ethylparaben.

The dry weight of the final preparation ranges between 12-15%, with particle size distribution of 98% particles between 100-300 microns.

The mass yield ranged from 88 to 92 %. The yield of expressed activity (A^{H}_{PenG} = 110 U/g ww; A^{H}_{PenG}= 750 U/g dw) was equivalent to 47% with respect to the activity taken for immobilization.

The activity was determined by alkalimetric titration using 25mM Penicillin G potassium, 50mM sodium phosphate buffer pH 8.0, at 37°C. The amoxicillin synthetic activity (A^{S}_{Amox} =150U/g dw) was determined at reaction conditions using 40 mmol of 6-APA and 60 mmol of HPGMe.HCl, at pH 6.3 and 40°C. The reaction was carried out for 30 minutes, followed by HPLC analysis for activity determination.

### Method B

The procedure as described in Example 5 Method A was followed but variable concentration of acrylamide and N,N'-Methylenebisacrylamide were used (Table 5). Literature reports suggest the use of the two monomers in the ratio ranging from 2:1 to 20:1. The variation resulted in gel matrix of different pore size which would entrap protein molecules to different extent. Also the different ratio of acrylamide and N'N-methylenebisacrylamide affects the pore size and the catalytic activity of the resultant biocatalyst.

**Table 5:**

| Experiment No. | Concentration of Acrylamide (% w/v) | Concentration of N'N-methylenebisacrylamide (% w/v) | Ratio of acrylamide to N'N-methylenebisacrylamide | % Yield of activity |
|---|---|---|---|---|
| 1 | 10 | 0.55 | 18:1 | 4.2 |
| 2 | 9.5 | 0.5 | 19:1 | 54.4 |
| 3 | 9.5 | 0.5 | 18:1 | 6.0 |
| 4 | 9 | 0.47 | 19:1 | 54 |
| 5 | 8.6 | 0.57 | 15 :1 | 36 |
| 6 | 8.5 | 0.45 | 19 :1 | 92 |
| 7 | 8.0 | 0.4 | 20:1 | 59 |
| 8 | 8.0 | 0.5 | 16: 1 | 24.7 |
| 9 | 7.5 | 0.45 | 16 :1 | 29.3 |
| 10 | 7.5 | 0.55 | 15 : 1 | 50.3 |
| 11 | 7.6 | 0.4 | 19 :1 | 70.1 |

Based on the above results, for better enzyme activity, concentration of acrylamide and N'N-methylenebisacrylamide used for catalyst preparation was 8.35% (w/v) and 0.43% (w/v), respectively, which corresponds to the ratio of 19 :1 (Experiment No. 6).

### Method C.

### Modified preparation of polyacrylamide gel aggregates: Fermase NA^{™}150

The procedure as described in Example 5, Method B was repeated with 8.35% (w/v) of acrylamide and 0.43 % (w/v) N',N'-Methylenebisacrylamide except that varying concentration of Glutaraldehyde was used for cross linking. The concentration chosen varied from 1 to 5 millimole per milligram of protein. Optimum concentration of Glutaraldehyde was chosed based on strength of biocatalyst. Strength of biocatalyst was determined by subjecting the biocatalyst to mechanical stress. For this, 10 % of biocatalyst suspension is prepared in water and subjected to shaking with glass beads (5 mm diameter) in an orbital shaker at 25 °C at 70 RPM for 12 hrs. After stress treatment, the biocatalyst was checked for the residual enzyme activity and gravitational settling time. The results are summarized as in Table 6.

**Table 6**

| Experiment No. | Conc.of Glutaraldehyde (mM) | PA Activity U/ d.w | % Residual activity after mechanical stress | Settling time Before Stress (Ts) | Settling time After Stress (Ts) |
|---|---|---|---|---|---|
| 1 | 1.0 | 885 | 60 | 24 | 65 |
| 2 | 1.5 | 880 | 63 | 24 | 40 |
| 3 | 2.5 | 840 | 86 | 21 | 28 |
| 4 | 3.0 | 650 | 97 | 21 | 23 |
| 5 | 3.5 | 425 | 80 | 22 | 29 |
| 6 | 4.0 | 400 | 76 | 22 | 35 |
| 7 | 4.5 | 260 | 70 | 22 | 36 |
| 8 | 5.0 | 245 | 55 | 23 | 40 |

The biocatalyst with relatively less mechanical strength tend to break into finer particle and due to the buoyancy of these finer particles, the settling time increases. Hence, settling time is taken as a measure of the mechanical stability. Though the Experiment No.4 has less activity compared to its counterparts, due its stability it was selected as the best combination for further experimentation.

### Method D

It was suggested by literature to (Preparation of immobilized systems Edited by Ichiro Chibata,78-92,1978) use 0.75% - 1.0 % (w/v) Ammonium persulfate and 0.5% (v/v) TEMED for polymerization. The time required for complete polymerization was checked in each experiment. The results are discussed below in table 7.

**Table 7**

| Experiment | APS | TEMED | Time of polymerization |
|---|---|---|---|
| | (% w/v) | (% v/v) | Ts (s) |
| 9 | 0.75 | 0.5 | 50 |
| 10 | 0.75 | 0.6 | 45 |
| 11 | 1.0 | 0.5 | 45 |
| 12 | 1.0 | 0.6 | 35 |

### Example 6

### Evaluation of polyacrylamide gel aggregate (FERMASE NA ^{™} 150)

The catalyst was prepared by entrapment of the recombinant PA of *Achromobacter sp.CCM 4824* (rPA_{As}), expressed in *E.coli* BL21 CCM 7394. The gel entrapment into polyacrylamide was used as described in Example 5.

### Method A

The hydrolytic activity of catalyst was determined using alkalimetric assay (15 mM Penicillin G potassium, 0.1M sodium phosphate, pH 8.0 at 25 °C). The catalyst with the A^{H}_{PenG} of 27.5 U/g ww (13.5 % d.w., A^{H}_{PenG} of 204 U/g dw) was used in the synthetic experiments. In addition, the hydrolytic activity of the same catalyst was determined using alkalimetric assay at higher temperature (15 mM Penicillin G potassium, 0.1M sodium phosphate, pH 8.0 at 37 °C): the activity of catalyst was nearly two fold (A^{H}_{PenG} = 58 U/g ww, A^{H}_{PenG} = 430 U/g dw).

### Method B

The amoxicillin synthetic activity A^{S}_{Amox} of 192 U/g dw was determined at reaction conditions: 300 mmol 6-APA, 700 mmol HPGMe.HCl, pH 6.3, 25 °C.

The reactants in the course of amoxicillin synthesis were determined by HPLC method using column Luna 5µ C18, 150 x 2 mm (Phenomenex) thermostated at 28°C, mobile phase was 0.05M sodium phosphate buffer pH 3.1, containing 7.5% methanol at flow rate 0.2 ml/min. The peaks were monitored at 215 nm.

Preparation of sample: Sample of reaction mixture was diluted 200 times with mobile phase containing 0.05% Sodium lauryl sulfate, centrifuged (14 000 rpm, 10 min) and injected (volume of 10 µl). The retention times in HPLC analysis of HPG, 6-APA, HPGMe and AMOX were 2.3, 3.2, 5.5 and 7.6 min, respectively. The Unit of Amoxicillin synthesis is one micromole of Amoxicillin formed per 1 min under the conditions of the reaction.

### Example 7.

### Preparation of polyacrylamide gel aggregate (FERMASE PA ^{™} 1500).

In order to compare the synthetic efficiency of the recombinant PA from *Achromobacter sp* CCM 4824 expressed in *E.coli* BL21 CCM 7394, (FERMASE NA^{™} 150), similar immobilized preparation was made from recombinant *E. coli* PGA (rPGA_{Ec}). The catalyst was prepared by entrapment of the rPGA_{Ec} by the similar method as in Example 6 with changes in the composition and enzyme loading. The hydrolytic activity of catalyst (15.7 % dw) was determined using alkalimetric assay (15 mM Penicillin G potassium, 0.1M sodium phosphate, pH 8.0 at 25 °C).The catalyst with A^{H}_{PenG} of 43.6 U/g ww (A^{H}_{PenG} of 278 U/g dw) was used for amoxicillin synthesis. In addition, the hydrolytic activity of the same catalyst was determined using alkalimetric assay at higher temperature (15 mM Penicillin G potasium, 0.1M sodium phosphate, pH 8.0 at 37°C): activity of the catalyst was nearly two fold (A^{H}_{PenG} = 81 U/g ww, or 516 U/g dw). The amoxicillin synthetic activity A^{S}_{Amox} of 54 U/g dw was determined at reaction conditions: 300 mmol 6- APA, 700 mmol HPGMe.HCl, pH 6.3, 25 °C as described in Example 6.

### Example 8

### Synthesis of Amoxicillin trihydrate (AMOX) from 6-APA (nucleophile) and HPGMeHCl (acyl donor) using FERMASE NA^{™} 150 and FERMASE PA^{™} 1500-Batch reaction

A jacketed reactor (volume of 25 ml) equipped with a sieve at bottom (125 µm, nylon cloth) and stirrer was filled with 9.4 ml demineralised water (25 °C), 376 mg of 6-APA (92% purity, 1.6 mmol of nucleophile) and pH was adjusted to 7.5 with 40% sodium hydroxide. Then, 766 mg of HPGMe.HCl (99% purity, 3.48 mmol of acyl donor) was added and pH was adjusted to 6.3 with 40% sodium hydroxide. After that, a suspension of HPGMe.HCl crystals was formed. Subsequently, the amount of 0.6 g (ww) of FERMASE NA ^{™} 150 was transferred into the reactor, stirring was started (t=0 min), and the temperature was kept at 25 °C. After about 60 minutes, viscosity of the suspension slightly decreased (HPGMe.HCl crystals were dissolved and crystals of AMOX were formed). The pH increased slightly from 6.3 to 6.4 and in 180th minute the pH was readjusted to 6.3 with 20% phosphoric acid.

After 200 minutes, the pH was 6.17 and conversion of 96.9% Wn was reached.

After 270 min the pH decreased to 5.85, the conversion was 99.1% Wn. The initial rate of AMOX synthesis (A^{S} _{Amox}) equalled 85 U/g dw of catalyst, the rate of HPG formation was 27 U/g dw of catalyst. The value of the ratio S/Hᵢₙᵢₜ was 3.15. The course of the conversion is shown in Figure 1.a.(IA/VI)

The same experiment was performed with FERMASE PA ^{™} 1500 in a reactor (as above) was filled with 9.4 ml of demineralised water (25 °C), 376 mg of 6-APA (92% purity, 1.6 mmol of nucleophile) and pH was adjusted to 7.5 with 40% sodium hydroxide. Then, 766 mg of HPGMeHCl (99% purity, 3.52 mmol of acyl donor) was added and pH was adjusted to 6.3 with 40% sodium hydroxide. Subsequently, the amount of 0.6 g (wet weight) of FERMASE PA^{™} 1500 was transferred into the reactor, stirring was started (t=0 min), and the temperature was kept at 25 °C. The pH slightly increased from 6.3 to 6.4 and in 160th minute the pH was readjusted to 6.3 with 20% phosphoric acid.

After 200 minutes, the pH was 6.2 and the conversion of 65.1% Wn was reached. After 270 min, the pH decreased to 6.2 and the conversion was 75.6 % Wn. The initial rate of AMOX synthesis (A^{S}_{Amox}) equalled 53.7 U/g dw of catalyst, the rate of HPG formation was 62.3 U/g dw of catalyst. The value of the ratio S/Hᵢₙᵢₜ was 0.86. The course of the conversion is shown in Figure 1.b.(IB/VI)

Final residual concentrations of reactants are shown in Table 8.

### Example 9

### Synthesis of AMOX from 6-APA and HPGMeHCl using FERMASE NA^{™} 150 and AMOX purification

A reactor (volume of 250 ml) equipped with a sieve at bottom (125 µm, nylon cloth) and stirrer was filled with 94 ml demineralised water (25 C), 3.028 g of 6-APA (92% purity, 12.88 mmole of nucleophile) and pH was adjusted to 7.5 with 40% sodium hydroxide. Then, 6.964 g of HPGMeHCl (99% purity, 32 mmole of acyl donor) was added and pH was adjusted to 6.3 with 40% sodium hydroxide. Subsequently, the amount of 6.0 g (ww) of FERMASE NA ^{™} 150 was transferred into the reactor, stirring was started (t=0 min), and the temperature was kept at 25 °C. The pH started increasing slowly and in 60th, 80th, and 100th minute, the pH was readjusted to 6.3 with H₃PO₄ phosphoric acid(20%). After 140 minutes, the pH was 6.01 and the conversion of 96.8% Wn was reached and the reaction was terminated. A^{S}_{Amox} equalled 167.2 U/g dw of catalyst, the rate of HPG formation was 55.9 U/g dw of catalyst. The value of the ratio S/Hᵢₙᵢₜ was 2.99. The course of the conversion is shown in Figure 2.a.(IIA/VI) Final residual concentrations of reactants are shown in Table 8

The reaction mixture was acidified to pH 1.8 and solution was separated from catalyst by filtration under mild vacuum. The catalyst was washed with small amount of water. The solution was clarified by filtration through the cellulose filtration desk under vacuum and cooled in ice-water bath. The pH of the clear solution was adjusted to 4.2, the solution was placed to refrigerator for 16 hours and crystals of amoxicillin trihydrate were left to form. The crystals were separated by filtration through the nylon cloth filter (125 µm), quickly washed with cold acetone (-18 °C) and dried out. 5.0 g of isolated amoxicillin trihydrate represented the yield of 92.5 %.

### Example 10

### pH-controlled synthesis of AMOX from 6-APA and HPGMeHCl using FERMASE NA 150 Acyl donor/nucleophile ratio 1.49

A similar reactor as in Example 8 was filled with 82 ml of demineralised water (25 °C), 5.64 g of 6-APA (92 % purity, 24.0 mmole of nucleophile) and pH was adjusted to 7.5 with 40%sodium hydroxide. Then, 7.84 g of HPGMeHCl (99% purity, 35.66 mmole of acyl donor) was added and pH was adjusted to 6.3 with 40%sodium hydroxide. Subsequently, the amount of 18.0 g (ww) of FERMASE NA ^{™} 150 was transferred into the reactor, stirring was started (t=0 min), and the temperature was kept at 25 °C. The pH was readjusted to the value of 6.3 at 30th and 80th minute of the reaction with phosphoric acid (20%).

After 240 min, the pH was 5.65 and the conversion reached 91.3 % Wn. A^{S}_{Amox} (0-20 min) equalled 75.6 U/g dw of catalyst and the rate of HPG formation was 17.8 U/g dw of catalyst. The value of the ratio S/Hᵢₙᵢₜ was 4.25. The final residual concentrations of reactants are shown in Table 8. The course of the conversion is shown in Figure 2.b.(IIB/VI)

### Example 11

### Synthesis of AMOX from 6-APA) and HPGMeHCl) using FERMASE NA^{™} 150 and AMOX purification - Fed batch reaction.

The reactor as in Example 8 was filled in with 188 ml of demineralised water (25 °C), 6.92 g of 6-APA (92% purity, 29.44 mmole of nucleophile) and pH was adjusted to 7.5 with 40% sodium hydroxide. Then, 6.964 g (the first portion) of HPGMeHCl (99% purity, 31.68 mmole of acyl donor) was added and pH was adjusted to 6.3 with 40% sodium hydroxide.40%. The solution was clear, HPGMeHCl was completely dissolved. Subsequently, the amount of 12.0 g (ww) of FERMASE NA ^{™} 150 were transferred into the reactor, stirring was started (t=0 min), and the temperature was kept at 25 °C. After 20 min of reaction, 6.964 g (the second portion) of HPGMeHCl (31.68 mmole) was added and pH was adjusted to 6.3 with 40% sodium hydroxide. At 40th minute of the reaction, the third portion (6.964 g) of HPGMeHCl (31.68 mmole) was added. There was a tendency of pH to decrease in the course of the reaction so that it was re-adjusted at 60th, 80th, and 110th minute back to the value of 6.3 with 40% sodium hydroxide.

After 140 min, the pH decreased to 5.67 and the conversion reached 98.7 % Wn. AMOX crystallization started at 40th minute of the reaction. A^{S}_{Amox} (0-20 min) equalled 123.0 U/g dw of catalyst and the rate of HPG formation was 20.5 U/g dw of catalyst. After addition of the second dose of HPGMeHCl, the rate of synthesis increased to 190.0 U/g dw of catalyst and after the third dose it equalled 143.0 U/g dw. The value of the ratio S/Hᵢₙᵢₜ was 6.0 (0 - 20 min), after the second and the third dose of acyl donor the value decreased to 2.28 and 0.96, respectively. The time course of the conversion is shown in Figure 3.(III/VI) Final residual concentrations of reactants are shown in Table 8. In 40th minute of the reaction, precipitation of the AMOX started and the suspension was becoming more viscous.

The reaction mixture was diluted twice and the suspension of the AMOX was removed under mild vacuum and stirring. Finally, the catalyst was repeatedly washed with small volumes of water. The volumes were combined (465 ml), cooled in ice-water bath and acidified with 10% HCl hydrochloric acid to a pH of 1.8. The pH of solution of AMOX (497 ml) was re-adjusted to 2.2. The solution was clarified by filtration through the cellulose filtration desk under vacuum and the filter was washed with water. The pH of clear solution (522 ml) was adjusted to 4.2 and crystals of AMOX were left to form in refrigerator for 16 hours. The crystals were separated by filtration through the nylon cloth filter (125 µm), quickly washed with cold acetone (-18°C) and dried out. 10.5 g of AMOX was obtained which represents the molar yield of 85 %. The residual concentrations of amoxicillin in mother liquor, catalyst and acetone correspond to 9.9, 3.1 and 0.6% of the isolated product, respectively.

### Example 12

### Synthesis of AMOX from 6-APA and HPGMeHCl using FERMASE NA^{™} 150 and AMOX purification - Fed batch reaction.

A similar reactor as in Example 11 was filled in with 78 ml of demineralised water (25 °C), 3.46 g of 6-APA (92% purity, 14.72 mmole of nucleophile) and pH was adjusted to 7.5 with 40% sodium hydroxide. Then, 3.482 g of HPGMeHCl (99% purity, 15.84 mmole of acyl donor) was added and pH was adjusted to 6.3 with 40% sodium hydroxide. The substrate solution was clear, HPGMeHCl was dissolved completely. Subsequently, the amount of 22.0 g (ww) of FERMASE NA ^{™} 150 was transferred into the reactor, stirring was started (t=0 min), and the temperature was kept at 25 °C. After 20 min of reaction, 3.482 g (the second portion) of HPGMeHCl (15.84 mmoles) was added and pH was adjusted to 6.3 with 40% sodium hydroxide. At 40th minute of the reaction, the third portion (1.738 g) of HPGMeHCl (7.92 mmoles) was added. There was a tendency of pH to decrease in the course of the reaction so that it was re-adjusted at 50^{th} and 80^{th} minute back to the value of 6.3 with 40% sodium hydroxide. Final residual concentrations of reactants are shown in Table 8.After 70 min, the pH decreased to 5.67 and the conversion reached 98.1 % Wn. A^{S}_{Amox} (0 - 20 min) equalled 71.3 U/g dw of catalyst and the rate of HPG formation was 23.5 U/g dw of catalyst. After addition of the second dose of HPGMeHCl, the rate of synthesis increased to 121.2 U/g dw of catalyst and after the third dose it decreased to 37.2 U/g dw. The value of the ratio S/Hᵢₙᵢₜ was 3.0 (0 - 20 min), after the second and the third dose of acyl donor the value decreased to 2.8 and 0.47, respectively. After 20 min of the reaction, crystallization of AMOX started and the suspension was becoming more viscous.

The product was diluted twice and AMOX was isolated in a similar way as in Example 11. 5.1 g of AMOX was obtained which represents the yield of 82.6 %.

### Example 13

### Synthesis of AMOX from 6-APA and HPGMeHCl using FERMASE NA ^{™}150-batch reaction with postponed acyl donor addition

A similar reactor as in Example 9, was filled in with 94 ml of demineralised water (25 °C), 3.46 g of 6-APA (92% purity, 14.72 mmole of nucleophile) and pH was adjusted to 7.5 with 40% NaOH. Then, 6.094 g of HPGMeHCl (99% purity, 27.72 mmole of acyl donor) was added and pH was adjusted to 6.3 with 40% sodium hydroxide. The mixture became turbid due to formation of crystals of HPGMe.HCl. Subsequently, the amount of 6.0 g (ww) of FERMASE NA^{™} 150 was transferred into the reactor, stirring was started (t=0 min), and the temperature was kept at 25 °C. After 60 min of the reaction, 1.567 g (the second portion) of HPGMe.HCl (99% purity, 7.13 mmole) was added and pH was adjusted to 6.3 with 40% sodium hydroxide. There was a tendency of pH to decrease in the course of the reaction so that it was re-adjusted at 90^{th} minute back to the value of 6.3 with 40% sodium hydroxide.

After 160 min, the pH was 6.32 and the conversion reached 96.6 % w.r.n.. A^{S}_{Amox} (0 - 20 min) equalled 124.8 U/g dw of catalyst and the rate of HPG formation was 30.2 U/g dw. After addition of the second dose of HPGMeHCl, the rate of synthesis increased to 190.0 U/g dw of catalyst. The value of the ratio S/Hᵢₙᵢₜ was 6.0 (0 - 20 min) and after the second dose of acyl donor the value was 2.28. The time course of the conversion is shown in Figure 4(IV/VI). Final residual concentrations of reactants are shown in Table 8. The product was diluted twice and AMOX was isolated in a similar way as in Example 11. 5.2 g of AMOX was obtained which represents the yield of 84.2 %.

**Table 8. Amoxicillin synthesis-summary of results**

| Example | RM volume | 6-APA (purity 92%) initial | HPGMeHC / 6-APA molar ratio | | Catalyst | | | Final reacti on time | Conversion degree | 6-APA residual | HPGMe residual | HPG residual | Amox isolated | Yield |
|---|---|---|---|---|---|---|---|---|---|---|---|---|---|---|
| (No.) | (ml) | (g) | Initial | total | (g ww) | U¹⁾/g of 6-APA | (U¹⁾/ml) | (min) | (%) | (mM) | (mM) | (mM) | (g) | (%) |
| 8 | 10 | 0.376 | 2.18 | 2.18 | 0.6 | 191 | *6.6* | 270 | 99.1 | 1.4 | 73 | 106 | - | - |
| 8 | 10 | 0.376 | 2.18 | 2.18 | 0.6 | 191 | *6.6* | 270 | 75.6 | 39.0 | 97 | 144 | - | - |
| 9 | 100 | 3.028 | 2.46 | 2.46 | 6.0 | 237 | *6.6* | 140 | 96.8 | 4.2 | 59 | 97 | 5.0 | 92.5 |
| 10 | 100 | 5.64 | 1.49 | 1.49 | 18.0 | 382 | *19.8* | 240 | 91.3 | 19.1 | 55 | 61 | - | - |
| 11 | 200 | 6.92 | 1.08 | 3.23 | 12.0 | 207 | *6.6* | 140 | 98.7 | 1.9 | 130 | 136 | 10.5 | 85.0 |
| 12 | 100 | 3.46 | 1.08 | 2.69 | 22.0 | 760 | *24.2* | 70 | 98.1 | 2.8 | 76 | 108 | 5.1 | 82.6 |
| 13 | 100 | 3.46 | 1.88 | 2.37 | 6.0 | 207 | *6.6* | 160 | 96.6 | 5.0 | 85 | 102 | 5.2 | 84.2 |

| | | | | | | | | | | | | | | |
|---|---|---|---|---|---|---|---|---|---|---|---|---|---|---|
| ¹⁾ A^{H}_{PenG} = 110 U/g ww; 750 U/g dw; pH 8, 37 °C | | | | | | | | | | | | | | |

### Example 14

### Scale-up of AMOX from 6-APA and HPGMeHCl using FERMASE NA 150 and AMOX purification: scale of 400 ml

Enzymatic coupling was performed by using a 1000 ml, jacketed cylindrical glass reactor with working volume of 400 ml, end fitted with 3 ports. Mixing was done by a three lobe paddle type impeller, with variable speed control. The reaction was carried out at a constant stirring of 300 +/-5 RPM.

6.9 g of 6-APA, equivalent to 80 mmoles and 27.84 g of HPGMeHCl, equivalent to 320 mmoles were stirred in the reactor, one after the other till dissolution by adjusting the pH between 6.4 to 7.4 by using 1:1molar ammonia. After adjusting the final pH to 6.3 and volume to 400 ml, FERMASE NA^{™} 150 was added to initiate the synthetic reaction. The enzyme charged was equivalent to a loading of 644 units of A^{H}_{PenG} as mentioned elsewhere in the document per gram of β- lactam nucleus.

Enzymatic coupling was carried out at 28 °C and pH was monitored through out the reaction. Samples were withdrawn at constant intervals and analyzed in HPLC. The reaction was terminated based on the % conversion of 6-APA with concomitant increase in AMOX. The enzyme was removed from the reaction mass and washed thrice with 50 ml of water, till all the precipitate is washed from the enzyme. The enzyme is stored at 5°C till further use. After removal of immobilized enzyme the pH of the solution was adjusted to 2 and clear solution was filtered through filter paper, thereafter the solution was cooled to 5°C and pH was adjusted to 6.3. The precipitate appeared was filtered off and the remaining mother liquor was subjected to a pH of 8 to 9. Second precipitate was filtered off. Both precipitates were kept for vacuum drying. The conversion of 6-APA to AMOX was around 92%. HPLC analysis of the AMOX revealed the purity of 98.50%.The molar yield of AMOX was 57.6 %

### Example 15

### Scale-up of synthesis of AMOX from 6-APA and HPGMeHCl using FERMASE NA^{™} 150 and AMOX purification: scale of 50 ml

Enzymatic coupling was performed by using a 250 ml, jacketed cylindrical glass reactor with working volume of 50 ml, end fitted with 3 ports. Mixing was done by a three lobe paddle type impeller, with variable speed control. The reaction was carried out at a constant stirring of 300 +/-5 RPM.

1.296 grams of 6-APA, equivalent to 120 mmoles and 3.48 grams of HPGMeHCl, equivalent to 320 mmoles were stirred in the reactor, one after the other till dissolution by adjusting the pH between 6.4 to 7.4 by using 1:1 M ammonia. After adjusting the final pH to 6.3 and volume to 50 ml, FERMASE NA ^{™} 150 was added to initiate the synthetic reaction. The enzyme charged was equivalent to a loading of 178 units of A^{H} _{PenG} as mentioned elsewhere in the document per gram of β-lactam nucleus.

Enzymatic coupling was carried out at 28°C and pH was monitored through out the reaction. Samples were withdrawn at constant intervals and analyzed in HPLC. The reaction was terminated based on the % conversion of 6-APA with concomitant increase in AMOX. The enzyme was removed from the reaction mass and the washed thoroughly thrice with 20 ml of water, till all the precipitate is washed from the enzyme. The enzyme is stored at 5°C till further use. After removal of immobilized enzyme the solution was cooled to 5°C and pH was adjusted to 6.3. The precipitate appeared was filtered off and the remaining mother liquor was subjected to a pH of 8 to 9. The precipitate appeared was filtered off. Both precipitates were kept for vacuum drying. The conversion of 6-APA to AMOX was around 91.8% HPLC analysis revealed the purity of AMOX to be 98.2% The molar yield of AMOX with respect to 6-APA charged was 57%.

### Example 16

### Scale-up of synthesis of AMOX from 6-APA and HPGMeHCl using FERMASE NA ^{™}150 and AMOX purification scale of 50 ml

Enzymatic coupling was performed by using a 250 ml, jacketed cylindrical glass reactor with working volume of 50 ml, end fitted with 3 ports. Mixing was done by a three lobe paddle type impeller, with variable speed control. The reaction was carried out at a constant stirring of 300 +/-5 RPM.

1.51 grams of 6-APA, equivalent to 140 mmoles and 3.48 grams of HPGMeHCl, equivalent to 320 mmoles were stirred in the reactor, one after the other till dissolution by adjusting the pH between 6.4 to 7.4 by using 1:1 M ammonia. After adjusting the final pH to 6.3 and volume to 50 ml, FERMASE NA ^{™} 150 was added to initiate the synthetic reaction. The enzyme charged was equivalent to a loading of 178 units of A^{H} _{PenG} as mentioned elsewhere in the document per gram of β- lactam nucleus.

Enzymatic coupling was carried out at 28°C and pH was monitored through out the reaction. Samples were withdrawn at constant intervals and analyzed in HPLC. The reaction was terminated based on the % conversion of 6-APA with concomitant increase in AMOX. The enzyme was removed from the reaction mass and the washed thrice with 20 ml of water, till all the precipitate is washed from the enzyme. The enzyme is stored at 5°C till further use. After removal of immobilized enzyme the solution was cooled to 5°C and pH was adjusted to 6.3. The precipitate appeared was filtered off and the remaining mother liquor was adjusted to a pH of 8 to 9. The second precipitate appeared was also filtered off. Both precipitates were kept for vacuum drying. The conversion of 6-APA to AMOX was around 94%. HPLC analysis revealed the purity of AMOX to be 98.54% and the molar yield of AMOX was 70%.

### Example 17

### Scale-up of synthesis of AMOX from 6-APA and HPGMeHCl using FERMASE NA ^{™} 150 and AMOX purification: scale of 100 ml

Enzymatic coupling was performed by using a 250 ml, jacketed cylindrical glass reactor with working volume of 100 ml, end fitted with 3 ports. Mixing was done by a three lobe paddle type impeller, with variable speed control. The reaction was carried out at a constant stirring of 300 +/-5 RPM. 2.16 grams of 6-APA, equivalent to 100 mmoles and 6.96 grams of HPGMeHCl, equivalent to 320 mmoles were stirred in the reactor, one after the other till dissolution by adjusting the pH between 6.4 to 7.4 by using 1:1 M ammonia. After adjusting the final pH to 6.3 and volume to 100 ml, FERMASE NA ^{™} 150 was added to initiate the synthetic reaction. The enzyme charged was equivalent to a loading of 178 units of A^{H} _{PenG} as mentioned elsewhere in the document per gram of β-lactam nucleus. Enzymatic coupling was carried out at 28°C and pH was monitored through out the reaction. Samples were withdrawn at constant intervals and analyzed in HPLC. The reaction was terminated based on the % conversion of 6-APA with concomitant increase in Amoxicillin. The enzyme was removed from the reaction mass and the washed thrice with 20 ml of water, till all the precipitate is washed from the enzyme. The enzyme is stored at 5°C till further use. After removal of immobilized enzyme the solution was cooled to 5°C and pH was adjusted to 6.3. The precipitate appeared was filtered off and the remaining mother liquor was adjusted to a pH of 8 to 9. The precipitate appeared was filtered off. Both precipitates were kept for vacuum drying. The conversion of 6-APA to AMOX was around 95% HPLC analysis revealed the purity of AMOX to be 98.48% and molar yield of AMOX was 74.9 %.

### Example 18.

### Repeated synthesis of AMOX from 6-APA and HPGMeHCl using FERMASE NA ^{™} 150 and AMOX purification: Enzyme recycling

Reaction was performed by using a 250 ml, jacketed cylindrical glass reactor with working volume of 100 ml, end fitted with 3 ports. Mixing was done by a three lobe paddle type impeller, with variable speed control. 3.88 grams of 6-APA, equivalent to 180 mmoles and 6.96 grams of HPGMeHCl, equivalent to 320 mmoles are stirred in the reactor, one after the other till dissolution by adjusting the pH between 6.4 to 7.4 by using.40% sodium hydroxide. After adjusting the final pH to 6.3 and volume to 100 ml, 15 grams ww of FERMASE NA ^{™} 150 (A^{H}_{Pen G}: 146 IU/g wet, 724 IU/ dry weight, A^{S}_{Amox} :45.7 Units per gram wet, 221 U/ gram dryweight) was added to initiate the synthetic reaction. The enzyme charged was equivalent to a loading of 567 units A^{H} _{PenG} as mentioned elsewhere in the document per gram of β-lactam nucleus.

Enzymatic coupling was carried out at 28°C and pH was monitored through out the reaction. Samples were withdrawn at constant intervals and analyzed in HPLC. The reaction was terminated based on the % conversion of 6-APA with concomitant increase in AMOX. The enzyme was removed from the reaction mass and the washed thrice with 50 ml of water, till all the precipitate is washed from the enzyme. The enzyme is stored at 5°C till further use. After removal of immobilized enzyme, the AMOx was isolated by following the downstream process as described in example 16. Enzyme was re-used for multiple cycles (data of 25 cycles are shown in Table 9)

**Table 9**

| **Cycle** **No** | **AMOX** | **% HPLC** | **%** **Molar** | **% Conv.** |
|---|---|---|---|---|
| **no.** | **In grams** | **Purity (dry)** | **Yield** | **(6-APA)** |
| 1 | 6.31 | 100.25 | 85 | 94.73 |
| 2 | 6.36 | 98.83 | 85.5 | 92.20 |
| 3 | 6.27 | 100.51 | 83.88 | 89.76 |
| 4 | 6.29 | 102.03 | 84.6 | 94.56 |
| 5 | 6.12 | 101.5 | 82.32 | 90.28 |
| 6 | 6.3 | 101.44 | 84.74 | 87.40 |
| 7 | 6.3 | 100.43 | 84.74 | 85.16 |
| 8 | 6.13 | 101.57 | 82.45 | 91.97 |
| 9 | 6.15 | 97.86 | 82.73 | 92.41 |
| 10 | 6.26 | 98.23 | 84.21 | 85.08 |
| 11 | 6.21 | 99.45 | 83.53 | 83.10 |
| 12 | 6.19 | 98.26 | 83.26 | 93.92 |
| 13 | 6 | 101.27 | 80.71 | 81.10 |
| 14 | 5.89 | 102.81 | 79.23 | 71.18 |
| 15 | 5.63 | 95.4 | 75.73 | 83.35 |
| 16 | 5.84 | 103.5 | 78.55 | 87.31 |
| 17 | 5.84 | 95.57 | 78.55 | 86.35 |
| 18 | 5.8 | 98.55 | 78 | 88.45 |
| 19 | 5.8 | 99.98 | 78 | 86.99 |
| 20 | 6.45 | 99.52 | 86.76 | 87.16 |
| 21 | 5.79 | 100.75 | 77.8 | 85.8 |
| 22 | 6.58 | 99.93 | 88.51 | 86.22 |
| 23 | 5.68 | 100.55 | 76.4 | 84.56 |
| 24 | 5.98 | 100 | 80.44 | 67.63 |
| 25 | 6.06 | 97.78 | 81.5 | 64.77 |

The average conversion of 6-APA was 85.66 and the average molar yield was 82.02

### Example 19

### Synthesis of Ampicillin trihydrate (AMP) from 6-APA and PGMeHCl using FERMASE NA 150.Acyl donor/nucleophile ratio 1.5 - Batch reaction at pH 5.9, 20 °C.

A jacketed reactor (volume of 250 ml) equipped with a sieve at bottom (125 µm, nylon cloth) and stirrer, was filled with 94 ml demineralised water (25 °C), 8.827 g of 6-APA (98 % purity, 40.0 mmole of nucleophile) and pH was adjusted to 7.5 with 40% sodium hydroxide.. Then, 12.473 g of PGMeHCl (97% purity, 60.0 mmole of acyl donor) was added and pH was adjusted to 5.9 with 40% sodium hydroxide. Subsequently, the amount of 6 g w/w of FERMASE NA ^{™} 150 was transferred into the reactor, stirring was started (t=0 min), and the temperature was kept at 20 °C. After about 70 minutes, the crystals of AMP were formed and viscosity of the suspension increased. The pH value of 5.9 was maintained in the course of the reaction.

After 360 minutes the conversion of 94.9 % Wn. was reached. The initial rate of AMP synthesis (A^{S}_{Amp}) equaled 351.1 U/g dw of catalyst, the rate of PG formation was 56.4 U/g dw of catalyst. The value of the ratio S/Hᵢₙᵢₜ was 6.0. The final residual concentrations of reactants are shown in Table 9. The course of the conversion is shown in Figure 5a(VA/VI).The reactants in the course of the ampicillin synthesis were determined by HPLC method using column Tessek 5µ C8, 250x4 mm (Tessek, Czech Republic) thermostated at 28 °C, mobile phase was 0.05M sodium phosphate buffer pH 6.0, containing 0.5 ml triethylamine per litre and 40 % methanol, flow 0.5 ml/min. The peaks were monitored at 215 nm. Preparation of sample: reaction mixture was diluted 400 times with mobile phase containing 0.05% Sodium lauryl sulfate, centrifuged (14 000 rpm, 10 min) and injected (volume of 10 µl). The retention times of 6-APA, PG, AMP, and PGMeHCl were 5.9, 6.85, 8.6 and 15.3, respectively.

The reaction mixture was diluted with 30 ml of water and catalyst was separated from the suspension of AMP on the sieve under vacuum. Crystals were separated from mother liquor by centrifugation. Enzyme catalyst was washed thrice with mother liquor and finally once with 20 ml of water. The crystals were suspended in mother liquor (washing solution), dissolved by acidification to pH 1.5 with 20% hydrochloric acid and solution was clarified by filtration through the cellulose filtration desk under vacuum and cooled in ice water bath. The pH of solution was adjusted to 4.2 and the suspension was stored in refrigerator for 16 hrs. Then the crystals were separated by centrifugation, washed with cold acetone (-18 °C) and dried out. 13.2 g of isolated AMP (purity 93 %) represented the yield of 76%.

### Example 20

### Synthesis of AMP from 6-APA and PGMeHCl using FERMASE NA 150 Acyl donor/nucleophile ratio 1.35 - Batch reaction at pH 5.9, 20 °C.

A similar reactor as in Example 19, was filled with 90 ml demineralised water (25 °C), 8.827 g of 6-APA (98 % purity, 40 mmoles of nucleophile) and pH was adjusted to 5.9 with 40% NaOH. Then, 11.226 g of PGMeHCl (97% purity, 54 mmoles of acyl donor) was added and pH was adjusted to 6.3 with 40% sodium hydroxide.. Subsequently, the amount of 10 g ww of FERMASE NA ^{™} 150 was transferred into the reactor, stirring was started (t=0 min), and the temperature was kept at 20 °C. After about 50 minutes, the crystals of AMP were formed and viscosity of the suspension increased. The pH value of 5.9 was maintained in the course of the reaction.

After 360 minutes the conversion of 94.6 % Wn. was reached. The initial rate of AMP synthesis (A^{S}_{Amp}) equalled 272.2 U/g dw of catalyst, the rate of PG formation was 25.8 U/g dw of catalyst. The value of the ratio S/Hᵢₙᵢₜ was 10.55. The final residual concentrations of reactants are shown in Table 9. The course of the conversion is shown in Figure.5.b.(VB/VI) The product was isolated similarly as in Example 19. 13.3 g of isolated AMP (purity 95.7 %) represented the yield of 82.5 %.

**Table 9. Ampicillin synthesis - summary of results**

| Example | RM volume | 6-AP A initial | PGM e/6-APA molar ratio | Catalyst | | | Final reaction time | Conversion degree | 6-APA residual | PGM e residual | PG residual | Amp product | Yield |
|---|---|---|---|---|---|---|---|---|---|---|---|---|---|
| No. | ml | gm | total | g ww | U¹⁾/g of 6-APA | U¹⁾/ ml | min | (%) | mM | mM | mM | gm | (%) |
| 19 | 100 | 8.8 27 | 1.50 | 6.0 | 76 | 6.6 | 360 | 94.9 | 20.5 | 97 | 100 | 13.20 | 76.0 |
| 20 | 100 | 8.8 27 | 1.35 | 10. 0 | 127 | 11.0 | 330 | 94.6 | 22.1 | 35 | 65 | 13.30 | 82.5 |

| | | | | | | | | | | | | | |
|---|---|---|---|---|---|---|---|---|---|---|---|---|---|
| ¹⁾ A^{H}_{PenG} = 110 U/g WW; 750 U/g dw; pH 8, 37°C | | | | | | | | | | | | | |

### Example 21

### Synthesis of cephalexin monohydrate (CEX) from 7-ADCA and PGMeHCl using FERMASE NA 150.Acyl donor/nucleophile ratio 1.5 - Batch reaction at pH 6.3, 25°C.

A similar reactor as in Example 19, was filled with 91 ml demineralised water (25 °C), 5.18 g of 7-ADCA (re-crystallized, 99 % purity, 24.0 mmoles of nucleophile) and pH was adjusted to 7.5 with 40% sodium hydroxide. Then, 7.49 mg of PGMeHCl (97% purity, 36.0 mmoles of acyl donor) was added and pH was adjusted to 6.3 with 40% sodium hydroxide. Subsequently, the amount of 9 g (ww) of FERMASE NA ^{™} 150 was transferred into the reactor, stirring was started (t=0 min), and the temperature was kept at 25 °C. After about 50 minutes, the crystals of CEX were formed and viscosity of the suspension increased. The pH slightly decreased and value of 6.3 was maintained.

After 210 minutes the conversion of 91.1% Wn was reached. The initial rate of CEX synthesis (A^{S}_{Cpx}) equaled 139 U/g dw of catalyst, the rate of PG formation was 21.5 U/g dw of catalyst. The value of the ratio S/Hᵢₙᵢₜ was 6.5. The final residual concentrations of reactants are shown in Table10. The course of the conversion is shown in Figure 6.a.(VIA/VI) The reactants in the course of the cephalexin synthesis were determined by HPLC method using column Tessek 5µ C8, 250x4 mm (Tessek, Czech Republic) thermostated at 28 °C, mobile phase was 0.05M sodium phosphate buffer pH 6.0 containing 0.5 ml triethylamine per L and 40 % methanol, flow 0.5 ml/min. The peaks were monitored at 215 nm.

Preparation of sample: reaction mixture was diluted 200 times with mobile phase containing 0.05% Sodium lauryl sulfate, centrifuged (14 000 rpm, 10 min) and injected (volume of 5 - 10 µl). The retention times in HPLC analysis of 7-ADCA, PG, CEX, and PGMeHCl were 5.85, 6.85, 8.2, and 15.3, respectively

### Example 22

### Synthesis of CEX from 7-ADCA and PGMeHCl using FERMASE NA 150_acyl donor/nucleophile ratio 1.34 - Batch reaction at pH 6.3, 25 °C

A similar reactor as in Example 19, was filled with 94 ml demineralised water (25 °C), 6.56 g of 7-ADCA (re-crystallized, 99 % purity, 30.3 mmoles of nucleophile) and pH was adjusted to 7.5 with 40% sodium hydroxide. Then, 8.41 g of PGMeHCl (97% purity, 40.5 mmoles of acyl donor) was added and pH was adjusted to 6.3 with 40% sodium hydroxide. Subsequently, the amount of 6 g (ww) of FERMASE NA ^{™} 150 was transferred into the reactor, stirring was started (t=0 min), and the temperature was kept at 25 °C. After about 70 minutes, the crystals of CEX were formed and viscosity of the suspension increased. The pH slightly decreased and value of 6.3 was maintained.

After 300 minutes the conversion of 88.5 % Wn. was reached. The initial rate of CEX synthesis (A^{S}_{Cpx}) equalled 208 U/g dw of catalyst, the rate of PG formation was 18.5 U/g dw of catalyst. The value of the ratio S/Hᵢₙᵢₜ was 11.3. The final residual concentrations of reactants are shown in Table 10. The course of the conversion is shown in Figure 6.b.(VI/VI)

**Table 10. Cephalexin synthesis - summary of results**

| Example | RM volume | 7-ADC A initial | PGMe / 6-APA molar ratio | Catalyst | | | Final reaction time | Conve rsion degree | 7-ADC A residual | PGM e residual | PG residual | Cpx final |
|---|---|---|---|---|---|---|---|---|---|---|---|---|
| No. | ml | gm | Total | g ww | U¹⁾/g of 6-APA | U¹⁾/ml | min | (%) | mM | mM | mM | mM |
| 21 | 100 | 5.18 | 1.50 | 9.0 | 193 | *9.9* | 210 | 91.1 | 21.3 | 29 | 77 | 210 |
| 22 | 100 | 6.56 | 1.34 | 6.0 | 102 | *6.6* | 300 | 88.5 | 34.3 | 47 | 53 | 260 |

| | | | | | | | | | | | | |
|---|---|---|---|---|---|---|---|---|---|---|---|---|
| ¹⁾ A^{H}_{PenG} = 110 U/g ww; 750 U/g dw; pH 8, 37 °C | | | | | | | | | | | | |

## Claims

1. A process for the synthesis of a semi-synthetic penicillin or cephalosporin antibiotic using Penicillin Acylase biocatalyst from *Achromobacter sp* CCM 4824 expressed in *Escherichia coli* BL21 CCM 7394 comprising:
a. chemically extracting Penicillin Acylase using solvent and chemical detergents or metal chelators for cell permeabilization;
b. purifying the soluble enzyme by fractional precipitation and chromatography;
c. immobilizing the said enzyme using acrylamide and N,N'-Methylenebisacrylamide in a ratio ranging from 15:1 to 20:1 at a temperature of -2 to 2°C;
d. stabilizing the immobilized enzyme by crosslinking with glutaraldehyde to obtain coarse particles with particle size ranging from 100 to 300 microns, preferably between 150 to 250 microns; and
e. enzymatically acylating the nucleophile with an activated acyl donor using the above immobilized Penicillin Acylase enzyme to obtain semi-synthetic penicillin or cephalosporin antibiotic with high yield and purity.

2. The process as claimed in claim 1, wherein the said solvent is selected from toluene, ethylacetate, chloroform and butylacetate.

3. The process as claimed in 1 or claim 2, wherein said chemical detergents or metal chelators are selected from the group comprising EDTA, cetrimide, urea and sodium lauryl sulphate.

4. The process as claimed in any one of claims 1 to 3, wherein:
a. said chemical extraction utilizes the combination of chloroform 1 to 3% w/v and sodium lauryl sulphate 0.1 to 0.2% w/v; and/or
b. said chemical extraction is carried out at a temperature of 15 to 35°C, optionally at 28 to 30°C, for 6 to 12hrs; and/or
c. the pH of the extraction is maintained in the range of 5.5 to 9.0, optionally between 6.5 to 7.5.

5. The process as claimed in any one of claims 1 to 4, wherein:
a. said purification is carried out using ammonium sulphate treatment; or
b. said purification is carried out using ion-exchange chromatography.

6. The process as claimed in any one of claims 1 to 5, wherein said enzyme immobilization provides the enzyme in aggregated form, optionally wherein the aggregation of the enzyme protein is done using ammonium sulphate at an amount corresponding to 30 to 70% of saturation.

7. The process as claimed in any one of claims 1 to 6, wherein said enzyme immobilization process is carried out in sodium or potassium phosphate buffer.

8. The process as claimed in any one of claims 1 to 7, wherein said immobilization further comprising a step of secondary crosslinking with glutaraldehyde.

9. The process as claimed in any one of claims 1 to 7, wherein said glutaraldehyde is used in a concentration of 2mM to 8mM, optionally between 4mM to 6mM.

10. The process as claimed in claim 8, wherein said immobilization further comprising a step of secondary crosslinking with glutaraldehyde at a concentration ranging from 0.3 to 0.5%(w/v).

11. The process as claimed in any one of claims 1 to 10, wherein said nucleophile is selected from 6- Aminopenicillianic acid or 7-Amino Deacetoxycephalosporanic acid.

12. The process as claimed in any one of claims 1 to 11, wherein said acyl donor is selected from D-p- hydroxyphenylglycine methyl ester or amide or D-phenylglycine methyl ester or amide.

13. The process as claimed in claimed in any one of claims 1 to 12, wherein said synthesis of antibiotics is effected at a pH ranging between 5.2 to 7.2.

14. The process as claimed in any one of claims 1 to 13, wherein the synthesis of antibiotic further comprising a step of removing a portion of semi-synthetic penicillin or cephalosporin antibiotic from the reaction mixture by lowering the pH of the said reaction mixture, optionally wherein said pH of the reaction mixture is adjusted by the addition of an acid, optionally conc. HCl.

15. The process as claimed in any one of claims 1 to 14, wherein:
a. said semi-synthetic penicillin antibiotic is Amoxicillin synthesized by acylation of 6-amino penicillanic acid with D-hydroxy phenyl glycine methyl ester, in a molar ratio ranging from 1.2 to 4.5, optionally between 1.5-1.8;
b. said semi-synthetic penicillin antibiotic is Ampicillin prepared by acylation of 6-amino penicillanic acid with D-phenyl glycine methyl ester, in a molar ratio ranging from 1.2 to 4.5, optionally between 1.3 to 1.7; or
c. said cephalosporin antibiotic is Cephalexin prepared by acylation of 7-Amino deacetoxy cephalosporanic acid with D-phenyl glycine methyl ester, in a molar ratio ranging 1.2 to 4.5,optionally between 1.5 to 1.7.

## Patentansprüche

1. Verfahren zur Synthese eines semisynthetischen Penicillin- oder Cephalosporin-Antibiotikums mit einem Penicillinacylase-Biokatalysator aus in *Escherichia coli* BL21 CCM 7394 exprimierter *Achromobacter sp.* CCM 4824, umfassend:
a. chemisches Extrahieren von Penicillinacylase mit einem Lösungsmittel und chemischen Detergentien oder Metallchelatoren zur Zellpermeabilisierung;
b. Reinigen des löslichen Enzyms durch fraktionierte Präzipitation und Chromatographie;
c. Immobilisieren des Enzyms mit Acrylamid und N,N'-Methylenbisacrylamid in einem Verhältnis im Bereich von 15:1 bis 20:1 bei einer Temperatur von -2 bis 2 °C;
d. Stabilisieren des immobilisierten Enzyms durch Vernetzung mit Glutaraldehyd zum Erhalt grober Partikel mit einer Partikelgröße im Bereich von 100 bis 300 µm, bevorzugt zwischen 150 bis 250 µm; und
e. enzymatisches Acylieren des Nukleophils mit einem aktivierten Acyldonor mittels dem vorstehend immobilisierten Penicillinacylase-Enzym zum Erhalt eines semisynthetischen Penicillin- oder Cephalosporin-Antibiotikums von hoher Ausbeute und Reinheit.

2. Verfahren nach Anspruch 1, worin das Lösungsmittel aus Toluen, Ethylacetat, Chloroform und Butylacetat ausgewählt ist.

3. Verfahren nach Anspruch 1 oder 2, worin die chemischen Detergentien oder Metallchelatoren aus der Gruppe ausgewählt sind, umfassend EDTA, Cetrimid, Harnstoff und Natriumlaurylsulfat.

4. Verfahren nach einem der Ansprüche 1 bis 3, worin:
a. sich die chemische Extraktion der Kombination aus Chloroform von 1 bis 3 % (G/V) und Natriumlaurylsulfat von 0,1 bis 0,2 % (G/V) zunutze macht; und/oder
b. die chemische Extraktion bei einer Temperatur von 15 bis 35 °C, gegebenenfalls bei 28 bis 30 °C, für 6 bis 12 h durchgeführt wird; und/oder
c. der pH der Extraktion im Bereich von 5,5 bis 9,0, gegebenenfalls zwischen 6,5 bis 7,5 aufrechterhalten wird.

5. Verfahren nach einem der Ansprüche 1 bis 4, worin:
a. die Reinigung mittels einer Ammoniumsulfatbehandlung durchgeführt wird; oder
b. die Reinigung mittels einer Ionenaustauschchromatographie durchgeführt wird.

6. Verfahren nach einem der Ansprüche 1 bis 5, worin die Enzymimmobilisierung das Enzym in aggregierter Form bereitstellt, worin die Aggregation des Enzymproteins gegebenfalls mit Ammoniumsulfat in einer Menge herbeigeführt wird, die 30 bis 70 % Sättigung entspricht.

7. Verfahren nach einem der Ansprüche 1 bis 6, worin das Enzymimmobilisierungsverfahren in Natrium- oder Kaliumphosphatpuffer durchgeführt wird.

8. Verfahren nach einem der Ansprüche 1 bis 7, worin die Immobilisierung ferner einen Schritt der sekundären Vernetzung mit Glutaraldehyd umfasst.

9. Verfahren nach einem der Ansprüche 1 bis 7, worin das Glutaraldehyd in einer Konzentration von 2 mM bis 8 mM, gegebenenfalls zwischen 4 mM bis 6 mM verwendet wird.

10. Verfahren nach Anspruch 8, worin die Immobilisierung ferner einen Schritt der sekundären Vernetzung mit Glutaraldehyd in einer Konzentration im Bereich von 0,3 bis 0,5 % (G/V) umfasst.

11. Verfahren nach einem der Ansprüche 1 bis 10, worin das Nukleophil aus 6-Aminopenicillansäure oder 7-Aminodesacetoxy-cephalosporansäure ausgewählt ist.

12. Verfahren nach einem der Ansprüche 1 bis 11, worin der Acyldonor aus D-p-Hydroxyphenylglycinmethylester oder -amid oder D-Phenylglycinmethylester oder -amid ausgewählt ist.

13. Verfahren nach einem der Ansprüche 1 bis 12, worin die Antibiotikasynthese bei einem pH im Bereich zwischen 5,2 bis 7,2 bewirkt wird.

14. Verfahren nach einem der Ansprüche 1 bis 13, worin die Antibiotikasynthese ferner einen Schritt zum Entfernen eines Teils von semisynthetischem Penicillin- oder Cephalosporin-Antibiotikum aus dem Reaktionsgemisch durch Senken des pH des Reaktionsgemischs umfasst, worin der pH des Reaktionsgemischs gegebenenfalls durch Zufügen einer Säure, gegebenenfalls konz. HCl, eingestellt wird.

15. Verfahren nach einem der Ansprüche 1 bis 14, worin:
a. das semisynthetische Penicillinantibiotikum Amoxicillin ist, das durch Acylierung von 6-Aminopenicillansäure mit D-Hydroxyphenylglycinmethylester in einem Molverhältnis im Bereich von 1,2 bis 4,5, gegebenenfalls zwischen 1,5 bis 1,8, synthetisiert wird;
b. das semisynthetische Penicillinantibiotikum Ampicillin ist, das durch Acylierung von 6-Aminopenicillansäure mit D-Phenylglycinmethylester in einem Molverhältnis im Bereich von 1,2 bis 4,5, gegebenenfalls zwischen 1,3 bis 1,7, hergestellt wird; oder
c. das Cephalosporin-Antibiotikum Cephalexin ist, das durch Acylierung von 7-Aminodesacetoxy-cephalosporansäure mit D-Phenylglycinmethylester in einem Molverhältnis im Bereich von 1,2 bis 4,5, gegebenenfalls zwischen 1,5 bis 1,7, hergestellt wird.

## Revendications

1. Procédé de synthèse d'un antibiotique à base de pénicilline ou de céphalosporine semi-synthétique en utilisant un biocatalyseur de pénicilline acylase issu de *Achromobacter sp* CCM 4824 exprimé dans *Escherichia coli* BL21 CCM 7394, comprenant :
a. l'extraction chimique de pénicilline acylase en utilisant un solvant et des détergents chimiques ou des chélateurs de métaux pour la perméabilisation cellulaire ;
b. la purification de l'enzyme soluble par précipitation fractionnée et chromatographie ;
c. l'immobilisation de ladite enzyme en utilisant de l'acrylamide et du N,N'-méthylènebisacrylamide selon un rapport allant de 15:1 à 20:1 à une température allant de -2 à 2°C ;
d. la stabilisation de l'enzyme immobilisée par réticulation par du glutaraldéhyde afin d'obtenir des particules grossières ayant une taille de particules allant de 100 à 300 microns, préférablement entre 150 à 250 microns ; et
e. l'acylation enzymatique du nucléophile par un donneur d'acyle activé en utilisant l'enzyme pénicilline acylase immobilisée ci-dessus afin d'obtenir un antibiotique à base de pénicilline ou de céphalosporine semi-synthétique avec un rendement et une pureté élevés.

2. Procédé selon la revendication 1, dans lequel ledit solvant est choisi parmi le toluène, l'acétate d'éthyle, le chloroforme et l'acétate de butyle.

3. Procédé selon la revendication 1 ou 2, dans lequel lesdits détergents chimiques ou chélateurs de métaux sont choisis dans le groupe constitué par l'EDTA, le cétrimide, l'urée et le laurylsulfate de sodium.

4. Procédé selon l'une quelconque des revendications 1 à 3, dans lequel
a. ladite extraction chimique utilise la combinaison de chloroforme de 1 à 3% p/v et de laurylsulfate de sodium de 0,1 à 0,2% p/v ; et/ou
b. ladite extraction chimique est effectuée à une température allant de 15 à 35°C, éventuellement de 28 à 30°C, pendant de 6 à 12 heures ; et/ou
c. le pH de l'extraction est maintenu dans la plage allant de 5,5 à 9,0, éventuellement entre 6,5 à 7,5.

5. Procédé selon l'une quelconque des revendications 1 à 4, dans lequel :
a. ladite purification est effectuée en utilisant un traitement par du sulfate d'ammonium ; ou
b. ladite purification est effectuée en utilisant une chromatographie d'échange d'ions.

6. Procédé selon l'une quelconque des revendications 1 à 5, dans lequel ladite immobilisation d'enzyme fournit l'enzyme sous forme agrégée, éventuellement où l'agrégation de la protéine enzymatique est effectuée en utilisant du sulfate d'ammonium en une quantité correspondant à de 30 à 70% de saturation.

7. Procédé selon l'une quelconque des revendications 1 à 6, dans lequel ledit procédé d'immobilisation d'enzyme est effectué dans un tampon de phosphate de sodium ou de potassium.

8. Procédé selon l'une quelconque des revendications 1 à 7, dans lequel ladite immobilisation comprend en outre une étape de réticulation secondaire avec du glutaraldéhyde.

9. Procédé selon l'une quelconque des revendications 1 à 7, dans lequel ledit glutaraldéhyde est utilisé selon une concentration allant de 2 mM à 8 mM, éventuellement entre 4 mM à 6mM.

10. Procédé selon la revendication 8, dans lequel ladite immobilisation comprend en outre une étape de réticulation secondaire avec du glutaraldéhyde selon une concentration allant de 0,3 à 0,5% (p/v).

11. Procédé selon l'une quelconque des revendications 1 à 10, dans lequel ledit nucléophile est choisi parmi l'acide 6-aminopénicillanique ou l'acide 7-aminodéacétoxycéphalosporanique.

12. Procédé selon l'une quelconque des revendications 1 à 11, dans lequel ledit donneur d'acyle est choisi parmi le méthylester ou amide de D-p-hydroxyphénylglycine ou le méthylester ou amide de D-phénylglycine.

13. Procédé selon l'une quelconque des revendications 1 à 12, dans lequel ladite synthèse d'antibiotiques est effectuée à un pH allant d'entre 5,2 à 7,2.

14. Procédé selon l'une quelconque des revendications 1 à 13, dans lequel la synthèse d'antibiotique comprend en outre une étape d'élimination d'une portion d'antibiotique à base de pénicilline ou de céphalosporine semi-synthétique à partir du mélange réactionnel en abaissant le pH dudit mélange réactionnel, éventuellement où ledit pH du mélange réactionnel est ajusté par l'addition d'un acide, éventuellement du HCl concentré.

15. Procédé selon l'une quelconque des revendications 1 à 14, dans lequel :
a. ledit antibiotique à base de pénicilline semi-synthétique est l'amoxicilline synthétisée par acylation d'acide 6-aminopénicillanique par du méthylester de D-hydroxyphénylglycine, selon un rapport molaire allant de 1,2 à 4,5, éventuellement entre 1,5-1,8 ;
b. ledit antibiotique à base de pénicilline semi-synthétique est l'ampicilline préparée par acylation d'acide 6-aminopénicillanique par du méthylester de D-phénylglycine, selon un rapport molaire allant de 1,2 à 4,5, éventuellement entre 1,3 à 1,7 ; ou
c. ledit antibiotique à base de céphalosporine est la céphalexine préparée par acylation d'acide 7-aminodéacétoxycéphalosporanique par du méthylester de D-phénylglycine, selon un rapport molaire allant de 1,2 à 4,5, éventuellement entre 1,5 à 1,7.
